# EUROPEAN PATENT SPECIFICATION

(11) **EP 3 880 843 B1**
(45) Date of publication and mention of the grant of the patent: **03.09.2025**
(21) Application number: 19801583.6
(22) Date of filing: 15.11.2019
(51) Int. Cl.: C12Q 1/682, C12Q 1/6841

(54) **METHOD FOR DETECTION OF RNA**
VERFAHREN ZUM NACHWEIS VON RNA
PROCÉDÉ DE DÉTECTION D'ARN

(30) Priority: 16.11.2018 GB 201818742
(43) Date of publication of application: 22.09.2021
(73) Proprietor: 10X Genomics, Inc., Pleasanton, CA 94588-3260 (US)
(72) Inventor: KÜHNEMUND, Malte, 17169 Solna (SE); NILSSON, Mats, 17893 Drottningholm (SE); LEE, Hao Zhe, 18145 Lidingö (SE)
(74) Representative: J A Kemp LLP
(86) International application number: PCT/EP2019/081496
(87) International publication number: WO 2020/099640

(56) References cited:
- WO-A1-2014/076214
- WO-A1-2016/016450
- WO-A1-2018/217905
- CHENGLIN WU ET AL: "RollFISH achieves robust quantification of single-molecule RNA biomarkers in paraffin-embedded tumor tissue samples", COMMUNICATIONS BIOLOGY, vol. 1, no. 1, 28 October 2018 (2018-10-28), XP055590333, DOI: 10.1038/s42003-018-0218-0
- HARRY M. T. CHOI ET AL: "Third-generation in situ hybridization chain reaction: multiplexed, quantitative, sensitive, versatile, robust", DEVELOPMENT, vol. 145, no. 12, 15 June 2018 (2018-06-15), GB, XP055590305, ISSN: 0950-1991, DOI: 10.1242/dev.165753

## Description

The present invention relates to the detection of RNA, especially mRNA, in a sample of cells. More particularly, the present invention relates to the localized detection of RNA, particularly mRNA, *in situ.* The invention combines the use of hybridization probes for the detection of the target RNA with signal amplification by rolling circle amplification (RCA), and relies upon hairpin probes which unfold upon hybridization to their target RNA to expose a sequence that can act as a binding site for a circular or circularisable probe, e.g. a padlock probe, and as a primer for rolling circle amplification (RCA) of a circular or circularized probe.

It is generally desirable to be able sensitively, specifically, qualitatively and/or quantitatively to detect RNA, and in particular mRNA, in a sample, including for example in fixed or fresh cells or tissues. This has utility in a number of areas, including notably in clinical diagnostic and prognostic assays, but also for examining RNA expression in different cell types to identify and categorise different cellular sub-types, particularly in the brain. It is thus particularly desirable to be able to detect an mRNA in a single cell. For example, in population-based assays that analyze the content of many cells, molecules in rare cells may escape detection. Furthermore, such assays provide no information concerning which of the molecules detected originate from which cells. Expression in single cells can vary substantially from the mean expression detected in a heterogeneous cell population. It is also desirable that single-cell studies may be performed with single-molecule sensitivity which allows the fluctuation and sequence variation in expressed transcripts to be studied. Fluorescence *in situ* hybridization (FISH) has been used to detect single mRNA molecules *in situ.* Although permitting determination of transcript copy numbers in individual cells, this technique cannot resolve highly similar sequences, so it cannot be used to study, for example, allelic inactivation or splice variation and cannot distinguish among gene family members.

Nonetheless, FISH has been used to quantify clinically relevant biomarkers in tumour specimens. In particular, single-molecule RNA FISH (smFISH) is a versatile assay that allows the detection of single RNA molecules with high specificity. In smFISH, a set of typically 30-50 oligonucleotides of 20 nucleotides, each directly conjugated to a single fluorophore are first hybridized to a complementary RNA target. Individual transcripts are then visualized as diffraction-limited spots using wide-field epifluorescence microscopy, and quantified (Raj et al, 2008, Nat Methods, 5, 877-879). Alternatively, smFISH probes may carry, instead of a direct conjugated fluorescent label, a universal 10-30 nucleotide long overhang sequence that can be detected by hybridization of universal fluorescently conjugated detection probes. The smFISH technique was recently used to quantify the expression and topographic distribution of two prominent breast cancer biomarkers and drug targets, epidermal growth factor receptor 2 (HER2) and estrogen receptor 1 (ER) in formalin-fixed, paraffin-embedded (FFPE) samples. In another study smFISH was used on mouse brain sections to quantify gene expression of cell type specific marker genes. In order to analyse several marker genes in the same tissue section, smFISH was performed sequentially by first adding a set of smFISH probes for 3 genes, each labelled with a different fluorescent reporter, and then fluorescently imaging the smFISH signals, removing the smFISH probes or the fluorescent labels and then hybridizing a different set of smFISH probes or probe labels for 3 other genes and imaging the smFISH signals. A major limitation of this approach, however, is that the smFISH signals are dim while background fluorescence is high in FFPE samples (with large variability depending on the tissue type, sample age, and fixation conditions). Hence, imaging at high magnification (60X-100X) is required. As a result, only a very small area of the sample is usually imaged (typically, 40-50 fields of view), thus limiting the ability to measure gene expression variability across a sample. This presents a disadvantage for analysis in cases where there can be a heterogeneity in expression as between different cells or regions of the tissue, for example in tumours, where it is becoming apparent that most cancer types harbour a high degree of intra-tumour heterogeneity and that the spatial distribution of cells expressing a biomarker might also represent a prognostic or predictive factor. It is therefore desirable to develop methods which are able not only to quantitatively determine the expression of an RNA molecule in a sample, and therefore provide a measure of its abundance, but which can also reflect the spatial distribution and heterogeneity of the RNA inside the sample.

In contrast to smFISH, other methods such as RNAscope (Wang F, et al., 2012, JMD 14, 22-29), rolling circle amplification (RCA) of padlock probes (Nilsson M, et al., 2002, Human mutation 19, 410-415) and single-molecule hybridization chain reaction (smHCR) (Crosetto et al, 2015, Nature reviews Genetics, 16, 57-66) involve one or more steps of signal amplification, which results in brighter fluorescence signals and higher signal-to-noise ratio (SNR). However, all of these methods have a number of drawbacks that limit their utility for the purpose of assessing gene expression variability in an *in situ* sample. Both smFISH and RNAscope require a large number of probes per target and the design of RNAscope probes is not open-source. In addition, the sensitivity of RNAscope is lower compared to smFISH. Padlock probe-based RCA assays provide advantages in terms of specificity and sensitivity and can be used to provide localised detection *in situ.* Whilst working well on DNA targets, the use of padlock probes directly on RNA targets is associated with problems. Techniques have been described where the RNA target is first converted to cDNA and then the cDNA is targeted by padlock probes and then amplified by RCA *in situ* (Larsson et al. 2010, Nat Methods). *In situ* sequencing (ISS) is an approach in which the padlock probes are either barcoded or the padlock probes are gap-filled using the cDNA as template, and then amplified by RCA *in situ* and subsequently sequenced directly in situ by applying next generation sequencing chemistry onto the tissue sections carrying RCA products (Ke, et al. 2013, Nat Methods). The conversion of RNA to cDNA and the subsequent targeting of the cDNA, however, limits the sensitivity of the method and is a major drawback; a recent study has reported direct detection of mRNA in single cells based on RCA, but the detection efficiency remains low (Deng et al., 2017, Chemical science 8, 3668-3675). Fluorescent in situ SEQuencing (FISSEQ) is another in situ sequencing approach in which RCA products (RCPs) are generated from self-circularized cDNA in a non-targeted fashion, and then sequenced in situ by SOLiD technology (Lee JH, et al, 2014, Science, 343, 1360-1363). However, the sensitivity of FISSEQ is also low and the method is biased towards abundant transcripts. Lastly, smHCR is limited by low specificity due to the fact that smHCR hairpins can bind non-specifically within the sample and become subsequently amplified. To destabilize non-specific binding, smHCR is typically performed under very stringent hybridization conditions, which however limits the number of RNA molecules that can be effectively detected (Choi HM, Beck VA, Pierce NA, 2014, ACS nano, 8, 4284-4294).

Thus improved methods are required to quantify gene expression by detecting RNA with high sensitivity and specificity, especially *in situ,* and in a manner which allows spatial distribution to be determined. The present invention is directed to addressing this need, and the disadvantages of the methods discussed above.

A method has been developed which combines the versatility and sensitivity of smFISH with the signal amplification of RCA. As is well known in the art, RCA results in the generation of a long concatemeric nucleic acid product which comprises multiple tandemly repeated complementary copies of a circular RCA template, such as a circularized padlock probe, or other circular probe. Such a RCA product (RCP) coils into a bundle, or "blob", of DNA which may readily be detected, for example by the hybridization to the RCP of labeled detection probes, and visualized by microscopy or flow cytometry. In particular, by harnessing the signal generation mechanism and amplification afforded by RCA, the method of the present invention allows RNA targets to be visualized and quantified at single cell resolution across whole tissue sections, a much wider sample area than has up to now been possible with smFISH, while using significantly reduced numbers of probes as compared with standard smFISH. Furthermore, by using hairpin probes which open on binding to the target RNA, and which can then be targeted by padlock probes (or other, e.g. circular, probes), firstly the need for the RNA to be converted into cDNA can be avoided, and secondly the frequency of RCA reactions triggered by non-specific binding of the padlocks or other probes is significantly reduced, as the hairpin probe is only accessible for binding to the padlock or other probe when the hairpin probe has specifically bound to its target sequence and unfolded.

According to the present method, the RNA is targeted first with one or more hairpin probes which unfold upon binding to their target RNA sequence to reveal a 3' end region which is capable firstly of binding to a padlock probe, or other circular or circularisable probe and if necessary templating its ligation to circularize the probe, and secondly of priming a subsequent RCA reaction of the circularized/circular probe. The 3' end region of the hairpin probe which is exposed upon target binding is then targeted with a padlock (or other) probe. Hybridization of a circularisable probe, such as a padlock probe, to the hairpin probe allows circularization of the probe by ligation of the ends of the probe. The circularized padlock or other probe, or other circular probe, is then subjected to RCA and a RCP is detected. The RCP is localized to the target RNA since it is an extension of the hairpin probe, which remains hybridized to the target RNA. Although particularly suited to *in situ* applications, the method may be used more generally, including in homogenous, or "in solution" based formats.

Accordingly, a first aspect the present disclosure, and present invention, can be seen to provide a method for detection of target RNA in a sample (including for example a sample of cells) comprising:
(a) contacting said sample with at least one target-specific hairpin probe comprising;
   (i) a first domain which comprises a first region of complementarity to a target sequence in said target RNA and is capable of hybridising to said target RNA;
   (ii) a second domain which is not capable of hybridising to the target RNA, which comprises a second region of complementarity to a cognate circular or circularisable probe, and which is capable of acting as a primer for an RCA reaction;
   wherein the second domain is at least partly comprised within a hairpin structure of the probe, such that the second domain is not capable of binding to said cognate circular or circularisable probe and/or priming an RCA reaction until released from said hairpin structure, and allowing said hairpin probe to hybridise to the target RNA, wherein hybridisation of said first domain to said target sequence causes the hairpin structure to open and release said second domain;
(b) contacting said sample with a circular or circularisable probe cognate for said hairpin probe, wherein the circular or circularisable probe comprises one or more regions complementary to the second region of complementarity in the second domain, and hybridising the circular or circularisable probe to the hairpin probe at said second domain, wherein if the probe is a circularisable probe, it comprises 3' and 5' end regions complementary to the second region of complementarity in the second domain such that said ends are hybridised in juxtaposition for ligation, directly or indirectly, to each other;
(c) if said probe is a circularisable probe, ligating, directly or indirectly, the ends of said circularisable probe to circularise the circularisable probe, thereby providing an RCA reaction template;
(d) performing an RCA reaction using the circular probe of (b) or the RCA template of (c), wherein said RCA reaction is primed by the second domain; and
(e) detecting the product of said RCA reaction.

In an embodiment, a set of hairpin probes may be used in step (a), said probe set comprising two or more hairpin probes, each specific for a different target sequence in said target RNA, wherein the first domain of each hairpin probe in the set comprises a region of complementarity to a different target sequence. This thus allows a multiplicity of circular or circularisable probes to become indirectly bound to the target RNA, by means of the multiple hairpin probes, and therefore for multiple RCA reactions to be performed, increasing the signal provided by the RCA product.

In one embodiment the circular or circularisable probe is a circularisable probe, and in particular it is a padlock probe. A circularisable probe such as a padlock probe may be provided in one or more parts, which may hybridise to the second domain in such a manner that the respective ends of the probe or probe parts are juxtaposed for ligation to one another, in order to circularise the probe.

In a particular embodiment the method is for localised *in situ* detection of target RNA in a sample.

Another aspect of the present disclosure and invention provides a hairpin probe for use in detecting a target RNA molecule, said probe comprising:
(i) a first domain which comprises a first region of complementarity to a target sequence in a target RNA and is capable of hybridising to said target RNA;
(ii) a second domain which is not capable of hybridising to the target RNA, which comprises a second region of complementarity to a cognate circular or circularisable probe (e.g. a padlock probe), and which is capable of acting as a primer for an RCA reaction;
wherein the second domain is at least partly comprised within a hairpin structure of the probe, such that the second domain is not capable of binding to said cognate circular or circularisable probe and/or priming an RCA reaction until released from said hairpin structure, and wherein the hairpin structure is such that hybridisation of said first domain to said target sequence causes the hairpin structure to open and release said second domain.

A still further aspect of the present disclosure and invention provides a probe set comprising two or more hairpin probes as defined herein, each probe being specific for a different target sequence in the same target RNA, wherein the first domain of each hairpin probe in the set comprises a region of complementarity to a different target sequence in the target RNA.

Also provided according to the present disclosure and invention is a kit comprising:
(i) a target-specific hairpin probe or a probe set as defined herein; and
(ii) a circular or circularisable probe (e.g. a padlock probe) cognate for said hairpin probe, or circular or circularisable probes (e.g. padlock probes) cognate for the hairpin probes in the probe set, each circular or circularisable probe comprising one or more regions complementary to the second region of complementarity in the second domain of said hairpin probe(s), wherein if the probe is a circularisable probe, it comprises 3' and 5' end regions complementary to the second region of complementarity in the second domain such that said ends can hybridise in juxtaposition for ligation, directly or indirectly, to each other.

The method of the invention relies upon the detection of a rolling circle amplification product (RCP) to detect the target RNA. The RCP is generated as a consequence of recognition by a circular or circularisable probe, such as a padlock probe, indirectly, of the target RNA; the circular or circularisable probe binds not to the target RNA itself, but to an intermediate hairpin probe which itself binds to the target RNA, and which upon binding the target unfolds to release, (e.g. expose) a binding site, and if appropriate a ligation template for the circularisable (e.g. padlock) probe. The circular probe or, once ligated to circularise it, the circularised probe, is subjected to RCA. By providing multiple (i.e. two or more) hairpin probes each targeting a different sequence in the target RNA molecule, multiple circular or circularisable probes may indirectly be hybridised to the target RNA, and multiple RCA reactions may thereby take place, to produce multiple RCPs, each localised to the target RNA molecule. RCPs may readily be detected and visualised, allowing a ready visualisation of the target RNA in the sample.

In the method of the invention, the hairpin probe comprises a domain (the second domain) which can act as both a binding site, and hence, if appropriate a ligation template, for the circular or circularisable probe and a primer for the RCA reaction. However, before the hairpin probe interacts with, i.e. hybridizes to, the target RNA molecule, the 3' end of the hairpin probe which comprises the primer sequence is protected within a hairpin structure of the hairpin probe, such that it is not available to hybridise to the circular or circularisable probe, or to prime a RCA reaction. By "capable of acting as a primer" is meant that the domain has a 3' end is capable of being extended by a polymerase enzyme, when hybridised to a template molecule (here the circular or circularised probe).

Following contact with a sample under conditions that allow the hairpin probe to hybridize with its target RNA molecule, the hairpin structure is able to open, or in other words unfold, to release the second domain which contains a binding site for the circular or circularisable probe, and a sequence which acts as an RCA primer. The term "release" is used broadly herein to indicate that the second domain is released from the hairpin structure i.e. the conformation of the probe changes so that the second domain is no longer contained in a hairpin structure. More particularly the second domain is made available to function. This includes in particular that the 3' end of the probe is available to act as a primer for RCA of the circular or circularised probe, once a circularisable probe has been ligated. Release may include that the second domain becomes accessible for binding of the circular or circularisable probe, or that binding of the circular or circularisable probe is facilitated. In this way it can thus be seen that the opening of the hairpin probe may create an overhang or a single-stranded region, that is a region of probe which is not hybridised to the target RNA.

The use of circular or circularisable probes is well known in the art in the context of RCA reactions. Thus preformed nucleic acid circles may be used as part of an RCA-based signal generation process. Accordingly, in one embodiment the circular probe may be a simple preformed circle. A circularisable probe comprises one or more linear oligonucleotides which may be ligated together to form a circle. A typical example of a circularisable probe is a padlock probe, and indeed a circularisable probe can be viewed as, or alternatively referred to as, a padlock probe. Padlock probes are well known and widely used and are well-reported and described in the prior art. Thus the principles of padlock probing are well understood and the design and use of padlock probes is known and described in the art. A padlock probe is typically a linear circularisable oligonucleotide which has free 5' and 3' ends which are available for ligation, to result in the adoption of a circular conformation. It is understood that for circularization (ligation) to occur, the padlock probe has a free 5' phosphate group. To allow the juxtaposition of the ends of the padlock probe for ligation, the padlock probe is designed to have at its 5' and 3' ends, regions of complementarity to its target sequence (in this case the second region of complementarity comprised in the second domain of the hairpin probe). These regions of complementarity thus allow specific binding of the padlock probe to its target sequence by virtue of hybridization to specific sequences in the target, and by the hybridization the ends of the padlock probe are brought into juxtaposition for ligation. The ligation may be direct or indirect. In other words, the ends of the padlock probe may be ligated directly to each other or they may be ligated to an intervening nucleic acid molecule/sequence of nucleotides. Thus the end regions of the padlock probe may be complementary to adjacent, or contiguous, regions in the second domain, or they may be complementary to non-adjacent (non-contiguous) regions of the second domain (in which case, for ligation to occur, the "gap" between the two ends of the hybridized padlock probe is filled by an intervening molecule/sequence). This may be done according to principles well known in the art, either by extending the hybridized 3' end of the padlock probe using a polymerase (with the second domain acting as a template for the extension reaction) or by hybridization of one or more gap oligonucleotides in between the hybridized 3' and 5' ends. Thus, the padlock probe may be provided in 2 more parts, and there may be more than one ligation reaction in order to circularize it (e.g. there may be a ligation junction at each end of a gap oligonucleotide, to ligate it respectively to the first "backbone" oligonucleotide of the padlock probe, which has two hybridized ends).

In particular embodiments the padlock probe does not have secondary structure, and more particularly does not comprise intramolecular double-stranded regions or stem-loop structures. However, dumbbell probes are an alternative type of circularisable probe which do have secondary structure. A dumbbell probe may be viewed as a particular sub-type of padlock probe. Dumbbell probes are described in Zhou et al., Nucleic Acids Research, 2010, 38(15), e156. The dumbbell probe comprises two stem-loop structures, joined stem to stem, wherein one of the "loops" is not closed, but is open with free 5' and 3' ends available for ligation to each other. This "open loop" functions as the target-binding domain of the probe. The closed loop functions simply as a spacer to join the end of the duplex (stem). In other words it can be seen as a padlock probe with a region of duplex formed between complementary sequences (regions) of the padlock. The region of duplex functions as a signalling domain to which an intercalating agent can bind. Thus the "open loop" of a dumbbell probe may comprise the regions of complementarity to the second domain of the hairpin probe, in other words it may comprise the 5' and 3' end regions complementary to the second region of complementarity in the second domain.

The respective 5' and 3' ends of the circularisable (e.g. padlock) probe, or parts thereof, are ligated together using the unfolded hairpin probe as a ligation template, thereby forming a circular template for the RCA reaction.

By "circularisable" it is meant that the oligonucleotide which makes up or constitutes the circularisable probe is in the form of a linear molecule having ligatable ends which may circularised by ligating the ends together directly or indirectly, i.e. to each other, or to the respective ends of an intervening ("gap") oligonucleotide or to an extended 3' end of the circularisable oligonucleotide.

A circular or circularisable probe is defined as cognate to a hairpin probe to mean that it is specific for that hairpin probe, or in other words that it is able to bind specifically to that (cognate) hairpin probe, or more particularly to the second domain thereof. Thus a cognate circular probe comprises a region of complementarity to a corresponding cognate complementary region (or binding site) in the second domain of hairpin probe. A cognate circularisable probe comprises, at its respective 3' and 5' ends, regions of complementarity to corresponding cognate complementary regions (or binding sites) in the second domain of the hairpin probe, which acts as the ligation template. Thus, in the case where the probe is circularisable, the second region of complementarity in the second domain of the hairpin probe comprises at least two sub-regions which are complementary to the 3' and 5' end regions of the circularisable (e.g. padlock) probe, and these may be adjacent where the ends of the circularisable probe are directly ligated to each other, or non-adjacent, with an intervening "gap" sequence, where indirect ligation is to take place. In other words, the second region of complementarity may be split.

Since the present method requires binding of the circular or circularisable probe to an unfolded hairpin probe, and if appropriate ligation thereon, to form the RCA template, the RCA reaction is unable to take place unless the circular or circularisable probe has hybridised to the unfolded hairpin probe i.e. unless the hairpin probe has hybridised to its target and opened. Thus, the RCA reaction is dependent upon the structure of the hairpin probe and therefore on presence of the RNA target sequence. In this way the specificity of the method may be improved.

The steps of the method may be carried out sequentially, or certain steps may be performed simultaneously. For example, the contacting of steps (a) and (b) may be carried out simultaneously in the sense that the hairpin probes and circular or circularisable probes may be added to the sample at the same time, but it will be understood that the respective hybridisations will occur sequentially, with the hairpin probe hybridising first to its target sequence, opening of the hairpin, and then the circular or circularisable probe hybridising to the opened hairpin. Similarly, the reagents for ligation may be added to the sample together with the circularisable probes. Thus, steps (b) and (c) may be performed simultaneously, although it will be understood that the circularisable (padlock) probes will hybridise to the released second domain of the hairpin probes, before the ligation can take place. However, in practice it may be desirable to include one or more washing steps in between one or more of the steps set out above. For example, following hybridisation of the hairpin probes, the sample may be washed one or more times before being contacted with the circular or circularisable probes and, if appropriate, reagents for ligation, and incubated to allow ligation to take place.

Washing steps may be used to improve specificity, for example by including one or more washing steps after hairpin probe hybridisation, and/or after the circular or circularisable probe hybridisation and/or ligation steps. This may readily be achieved in a solid phase or immobilised format by washing away any unbound and/or unligated reactants. Accordingly, in the absence of the RNA target, the circular or circularisable probe has no "binding target" (i.e. no unfolded hairpin probe), and hence is not immobilised by binding to its target, and may be washed away. In this way the RCA template is not available for the RCA reaction if the RNA target is absent.

Accordingly, the method may be conducted such that unbound circular or circularisable probe is removed before carrying out the RCA reaction of step (d). This may readily be achieved for example by carrying out the method in a solid phase format and washing after the step of circular or circularisable probe addition and hybridisation (step (b)). Alternatively or additionally washing, e.g. stringent washing, may be carried out after ligation step (c) to remove any unligated probes.

The method of the invention is for the detection of RNA. Whilst of particular applicability to mRNA, the method may be used for the detection of any RNA molecule present in a cell, including but not limited to viral RNA, tRNA, rRNA, small nuclear RNA (snRNA), small nucleolar RNA (snoRNA), microRNA (miRNA), small interfering RNA (siRNA), piwi-interacting RNA (piRNA), antisense RNA and non-coding RNA.

To detect miRNA or other small RNA molecules it may be that only one hairpin probe per target RNA molecule is used. For longer RNA molecules, and in particular mRNA, it may be advantageous to use a probe set comprising multiple hairpin probes each targeting a different target sequence in the target RNA molecule. In particular the different target sequences are non-overlapping. As noted above, multiple means two or more, particularly 3 or 4 or more, e.g. 2-50, 2-40, 2-30, 2-20, 2-10, 2-8, 2-6, or 2-5. In one representative embodiment, 3-6 hairpin probes per target RNA molecule could be used. In another representative embodiment, 20-50 hairpin probes could be used. The number could be between any of the lower and upper limit integers set out above, which are given by way of example only.

A target RNA is thus the RNA molecule that it is desired to detect, or in other words the analyte of the assay. The method of the invention may be performed in multiplex to detect two or more different target RNAs. For each different target RNA a different hairpin probe, or probe set, will be used. Thus, the sample may be contacted with multiple different hairpin probes, or probe sets, each specific for a different target RNA. In such a multiplex embodiment, the hairpin probes within each target-specific probe set are each specific for a different target sequence in the respective target RNA. A target sequence is the sequence in the target RNA which is bound by the hairpin probe. As will be described in more detail below, where a probe set of hairpin probes is used, such that multiple different target sequences are targeted, the circular or circularisable probes can be designed to report with respect to the target RNA, or with respect to the target sequence in the target RNA. In other words the signal generated by the circular or circularisable probe may be unique to a particular target sequence or it may be common to all the probes in a probe set, such that it is common for all the target sequences bound by the hairpin probes within a probe set, but distinguishable between different probe sets directed to different target RNAs. (The circular or circularisable probes for use with a given probe set may thus be common or unique for the hairpin probes of that set).Thus, in certain embodiments, a target sequence may itself be an analyte of the assay, for example if is desired to detect a particular target sequence in the target molecule, for example for the detection of splice variants or isoforms.

The term "detecting" is used broadly herein to include any means of determining the presence of the target RNA (i.e. if it is present or not) or any form of measurement of the target RNA. Thus "detecting" may include determining, measuring, assessing or assaying the presence or absence or amount or location of target RNA in any way. Quantitative and qualitative determinations, measurements or assessments are included, including semiquantitative. Such determinations, measurements or assessments may be relative, for example when two or more different target RNAs in a sample are being detected, or absolute. As such, the term "quantifying" when used in the context of quantifying a target RNA(s) in a sample can refer to absolute or to relative quantification. Absolute quantification may be accomplished by inclusion of known concentration(s) of one or more control RNAs and/or referencing the detected level of the target RNA with known control RNAs (e.g. through generation of a standard curve). Alternatively, relative quantification can be accomplished by comparison of detected levels or amounts between two or more different target RNAs to provide a relative quantification of each of the two or more different RNAs, i.e., relative to each other.

In one embodiment the method may be for the localised detection of target RNA. "Localised" detection means that the signal giving rise to the detection of the RNA is localised to the RNA, in this case the RCP is localised to the target RNA. The RNA may therefore be detected in or at its location in the sample. In other words the spatial position (or localization) of the RNA within the sample may be determined (or "detected"). This means that the RNA may be localised to, or within, the cell in which it is expressed, or to a position within the cell or tissue sample. Thus "localised detection" may include determining, measuring, assessing or assaying the presence or amount and location, or absence, of RNA in any way. In a particular embodiment, the method and probes may be used for the localised, particularly *in situ,* detection of mRNA. More particularly, the method and probes may be used for the localised, particularly *in situ,* detection of mRNA in a sample of cells.

As used herein, the term *"in situ"* refers to the detection of a target RNA in its native context, i.e. in the cell or tissue in which it normally occurs. Thus, this may refer to the natural or native localization of a target RNA. In other words, the RNA may be detected where, or as, it occurs in its native environment or situation. Thus, the RNA is not moved from its normal location, i.e. it is not isolated or purified in any way, or transferred to another location or medium etc. Typically, this term refers to the RNA as it occurs within a cell or within a cell or tissue sample, e.g. its native localization within the cell or tissue and/or within its normal or native cellular environment. In particular, *in situ* detection includes detecting the target RNA within a tissue sample, and particularly a tissue section. In other embodiments the method can be carried out on a sample of isolated cells, such that the cells are themselves are not *in situ.*

In other embodiments, the detection is not localized, or not *in situ.* In still other embodiments, the method can be carried out in solution. In particular the RNA can be in solution. Thus, for example, the method can be performed on a sample comprising isolated RNA.

The sample may be any sample which contains RNA. It may contain RNA from a cellular source. In an embodiment, the sample may be a sample of cells, or a sample containing cellular material, or a sample derived from a sample of cells. All biological and clinical samples are included, e.g. any cell or tissue sample of an organism, or any body fluid or preparation derived therefrom, as well as samples such as cell cultures, cell preparations, cell lysates etc. Environmental samples, e.g. soil and water samples or food samples are also included. The samples may be freshly prepared or they may be prior-treated in any convenient way e.g. for storage.

The sample may be a biological sample, which may contain any viral or cellular material, including all prokaryotic or eukaryotic cells, viruses, bacteriophages, mycoplasmas, protoplasts and organelles. Such biological material may thus comprise all types of mammalian and non-mammalian animal cells, plant cells, algae including blue-green algae, fungi, bacteria, protozoa etc. The cells may be for example human cells, avian cells, reptile cells etc., without limitation. The sample may be pre-treated in any convenient or desired way to prepare for use in the methods of the invention. The sample may further comprise an isolated nucleic acid or isolated RNA, or indeed synthetic RNA.

For localised *in situ* detection the sample may be any sample of cells in which a RNA molecule may occur, to the extent that such a sample is amenable to localized *in situ* detection. Typically, the sample may be any biological, clinical or environmental sample in which the RNA may occur, and particularly a sample in which the RNA is present at a fixed, detectable or visualizable position in the sample. The sample will thus be any sample which reflects the normal or native (*"in situ"*) localization of the RNA, i.e. any sample in which it normally or natively occurs. Such a sample will advantageously be or comprise a cell or group of cells such as a tissue. Particularly preferred are samples such as cultured or harvested or biopsied cell or tissue samples in which the RNA may be detected to reveal the qualitative nature of the RNA, i.e. that it is present, or the nucleotide sequence of the RNA or the presence and/or identity of one or more nucleotides in the RNA, and localization relative to other features of the cell. The sample of cells may be freshly prepared or may be prior-treated in any convenient way such as by fixation or freezing. Accordingly, fresh, frozen or fixed cells or tissues may be used, e.g. FFPE tissue (Formalin Fixed Paraffin Embedded). The sample may comprise any cell type that contains RNA including all types of mammalian and non-mammalian animal cells, plant cells, algae including blue-green algae, fungi, bacteria, protozoa etc.

Representative samples thus include clinical samples, e.g. whole blood and blood-derived products, blood cells, tissues, biopsies, as well as other samples such as cell cultures and cell suspensions, etc.

A representative sample for *in situ* detection may comprise a fixed tissue section, a fresh frozen tissue or a cytological preparation comprising one or more cells. The sample may be permeabilized to render the RNA accessible. Appropriate means to permeabilize cells are well known in the art and include for example the use of detergents, e.g. appropriately diluted Triton X-100 solution, e.g. 0.1% Triton X-100, or Tween, 0/1% Tween, or acid treatment e.g. with 0.1M HCI. Permeabilization of tissue samples may also comprise treatment of the sample with one or more enzymes, e.g. pepsin, proteinase K, trypsinogen, or pronase, etc. Also, microwave treatment of the sample may be carried out as described in the art.

The sample may also be treated to fix RNA contained in the cells to the sample, for example to fix it to the cell matrix. Such procedures are known and described in the art. For example, in the field of *in situ* hybridization, reagents are known for fixing mRNA to cells. In particular, 5' phosphate groups in the RNA may be linked to amines present on proteins in the cellular matrix via EDC-mediated conjugation (EDC: 1-ethyl-3-(3-dimethylaminopropyl) carbodiimide), thus helping to maintain the localization of the RNA relative to other cellular components. Such a technique has previously been described in relation to microRNAs and their detection via in situ hybridization (Pena et al. (2009) Nat. Methods, 6(2): 139-141).

The hairpin probe comprises a hairpin structure which at least partially harbours the second domain of the probe. In particular, the second domain may be fully contained within the hairpin structure. A hairpin structure may also be referred to as a hairpin-loop, or stem-loop structure, and these terms are interchangeable. The hairpin probe comprises at least one hairpin structure, and typically it may comprise a single hairpin structure. A hairpin occurs when two regions of the same strand, usually complementary in nucleotide sequence when read in opposition directions, base-pair to form a duplex that ends in an unpaired, i.e. single-stranded, loop. Thus, the hairpin probe contains two regions of mutual complementarity which hybridise to one another to form a stem-loop structure.

The regions of mutual complementarity occur at, or towards the respective 5' and 3' ends of the hairpin probe. In one embodiment the first mutually complementary region lies internally of the 5' end of the probe, and the second mutually complementary region lies at the 3' end of the probe, such that a hairpin structure is formed having a single-stranded region at the 5' end of the probe. In another embodiment both the mutually complementary regions lie at the 5' and 3' ends of the probe, such that both end regions form part of the stem.

Thus, according to the present methods and probes, the hairpin probe may be an oligonucleotide, wherein at least part of the region at the 5' end of the oligonucleotide is capable of hybridising to a region at the 3' end of the oligonucleotide, such that the oligonucleotide forms a hairpin structure.

Accordingly, in one embodiment the hairpin structure comprises 5' to 3':
(i) a single stranded region at the 5' end;
(ii) a first stem strand region;
(iii) a single stranded loop region; and
(iv) a second stem strand region at the 3' end.

In another embodiment, the 5' single stranded region may be omitted.

The hairpin probes may be configured in different ways such that the first and second domains may lie, fully or partially, within the loop or the stem region of the hairpin structure or both. It will be understood in this respect that the first and second domains are separate in the probe, and not overlapping and hence cannot both lie simultaneously in the exact same region of the hairpin probe.

In a particular embodiment the first, target-binding, domain may lie partially in a 5' single stranded region.

Thus in one embodiment the second domain of each member of the probe set comprises a sequence that is capable of hybridising to at least a portion of the region of complementarity to the target sequence present in the first domain, such that parts of said first and second domains are comprised within the stem of the hairpin structure. In one such representative embodiment of a hairpin probe, the second domain may lie partially in the stem and partially in the loop. The first domain may lie partially in a 5' single stranded region and partially in the stem. Thus a first strand of the stem may contain part of the first domain and the second strand of the stem may contain part of the second domain.

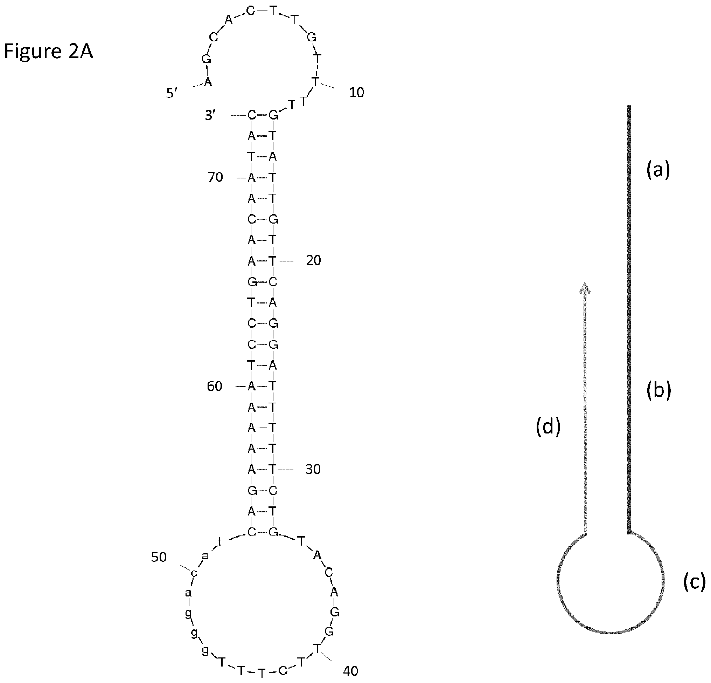

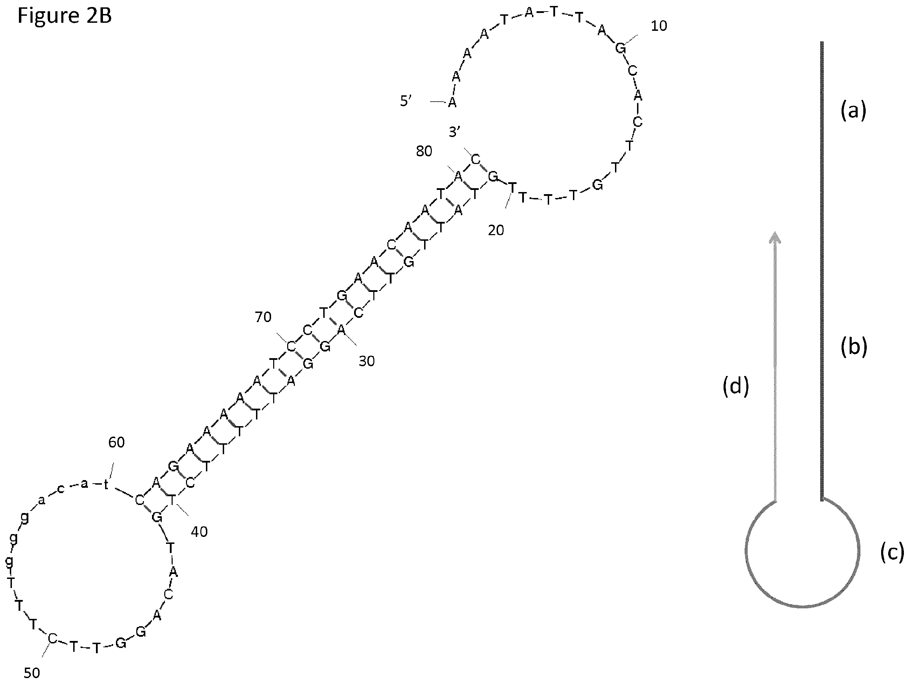

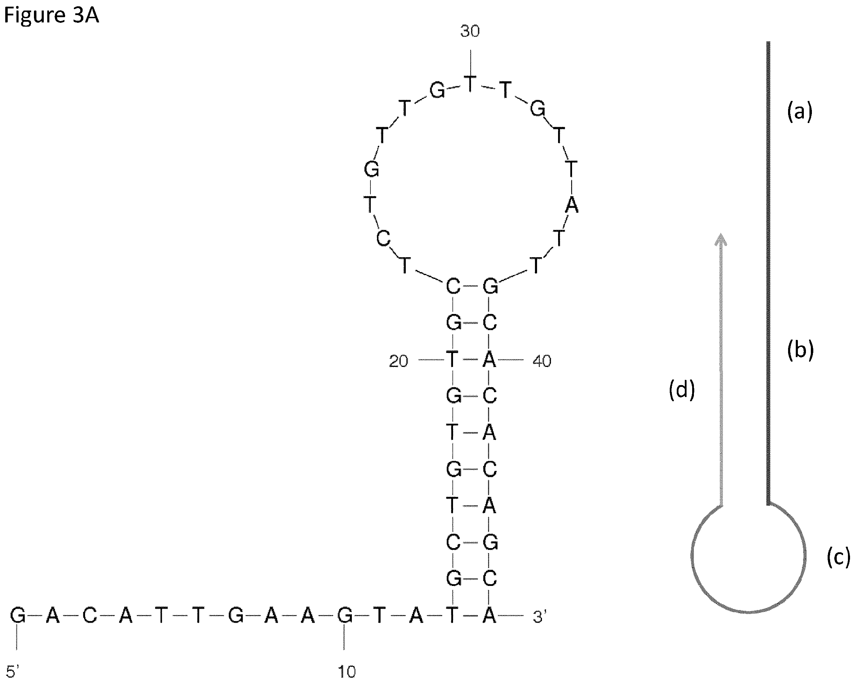

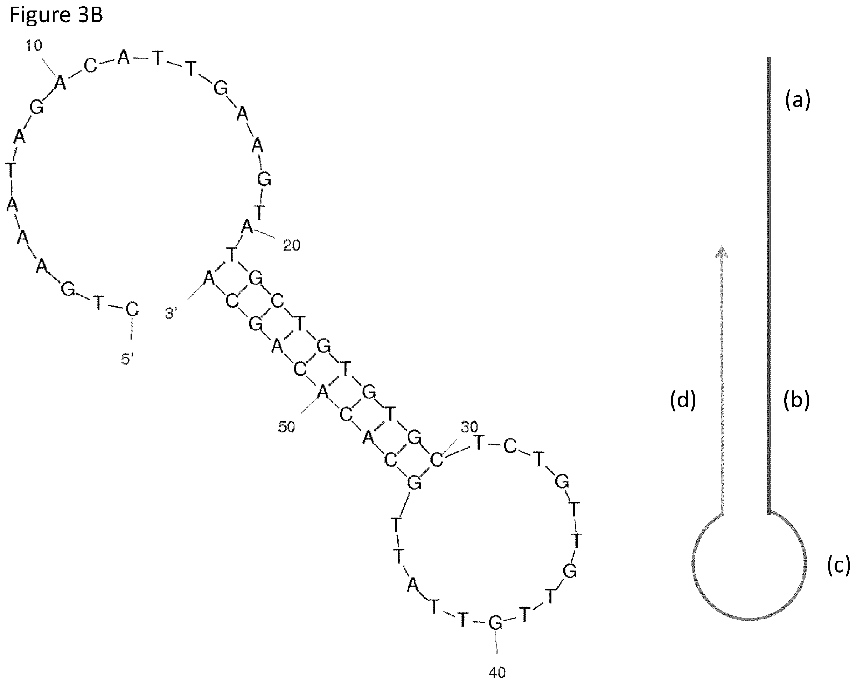

In such an embodiment the first domain comprising the first region of complementarity to a target sequence may comprise all or a portion of the 5' single stranded region and at least a portion of the first stem strand region of the hairpin probe. The single stranded region, or a part thereof, may act as a toehold for binding of the target RNA. Such a configuration is depicted in Figure 2, and in Figure 3. The first domain may comprise at least a part of the 5' single stranded region and the first stem strand region. Thus, in this embodiment, target RNA binds initially to the toehold of the probe. This causes the stem of the hairpin to open, to expose the remainder of the first domain to allow the probe to bind to the first domain. This opening exposes the second domain in the second stem strand and loop structure. Since the second domain lies in both the stem and the loop, the second region of complementarity is not accessible for the circular or circularisable probe binding until the hairpin has been opened. In this respect the skilled person knows how to design regions of complementarity to favour one hybridisation over another, for example based on length of the stem, GC content of stem and/or loop etc, mismatches in the binding sites (complementary regions) etc. Thus, the hairpin probe and its cognate circular or circularisable probe may be designed so that the circular or circularisable probe is unable to hybridise until the hairpin has been opened by target binding. Alternatively, or additionally, in this embodiment, a washing step may be performed to remove hairpin probes which have not hybridised to target RNA, before circular or circularisable probes are added.

It will be understood that in such a configuration the second domain is at least partially complementary to the first domain, which contains the target binding site. Thus, the second domain can be seen to comprise a region of homology (or sequence identity) to the target sequence. The circular or circularisable probe which is cognate to that hairpin probe thus reflects the target sequence of the hairpin probe (more specifically, the region of complementarity in the circular or circularisable probe which is complementary to the second domain is also complementary to the target sequence in the target RNA). In effect this region of complementarity in the circular or circularisable probe to the second domain (more specifically the region in the second domain which is complementary to the target binding site in the first domain) can function as a reporter domain in the circular or circularisable probe, since it reflects the target sequence of the cognate hairpin. Reporter domains are discussed further below.

Any undesired hybridisation of the circular or circularisable probe to the target RNA can be minimised by appropriate design of reagents and/or reaction conditions, for example by taking advantage of differential stability of intra- versus intermolecular hybrids, differences in hybrid lengths and possible introduction of mismatches to destabilise undesired hybrids.

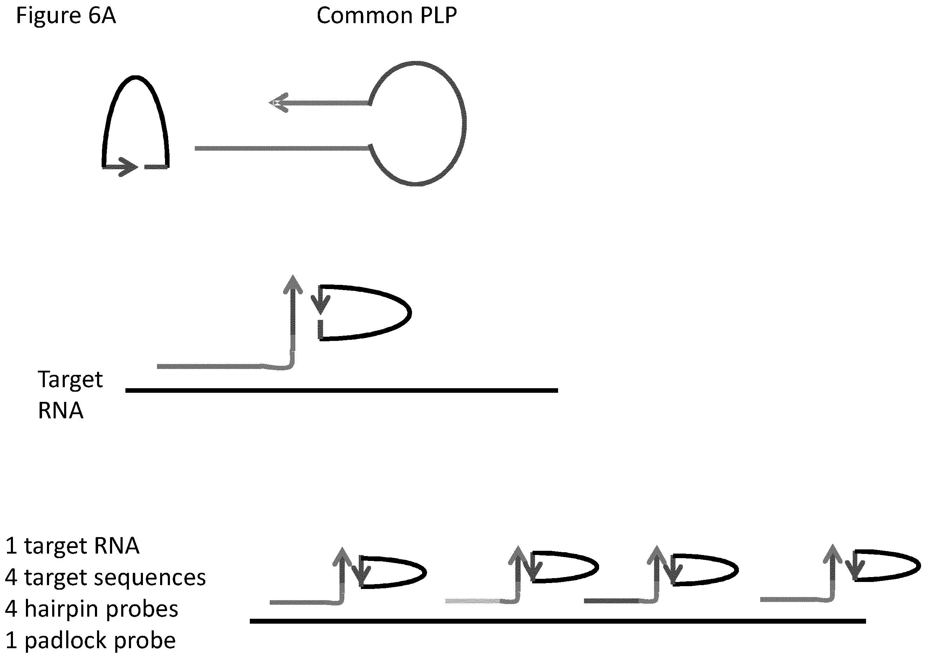

In a modification of such an embodiment, the second domain may lie only within the loop of the hairpin structure. Accordingly, in a second representative embodiment of a hairpin probe, the first domain comprises all or a portion of the 5' single stranded region and at least a portion of the first stem strand region of the hairpin probe (e.g. all of the first stem strand region). The single stranded region, or a part thereof, may act as a toehold for binding of the target RNA, as described above, and binding of the target RNA to the first domain causes the hairpin to open. The second domain comprises all or, more preferably, a part of the loop region. Opening of the hairpin may facilitate binding of the circular or circularisable probe to the second domain. Whilst it may be possible, in some circumstances, for the probe to bind to the loop of an un-opened hairpin probe (e.g. in the absence of target, or to an unhybridised hairpin probe), RCA of such a circular or circularised probe cannot take place, since the 3' end of the hairpin probe is not available to hybridise to the circular or circularised probe to prime the RCA. Such a probe design is depicted in Figure 6A.

In another, third, representative embodiment of a hairpin probe, the first domain may lie in the loop region of the hairpin structure, and the probe may be designed such that hybridisation of the hairpin probe to its target sequence causes the hairpin probe to open, to release the stem regions from their duplex. In such a configuration the hairpin probe may take the form of a stem-loop structure without a single stranded end region, i.e. where both the 5' and 3' end regions of the probe are hybridised together in the stem. Thus, in such an embodiment the hairpin structure comprises 5' to 3':
(i) a first stem strand region at the 5' end;
(ii) a single stranded loop region; and
(iii) a second stem strand region at the 3' end.

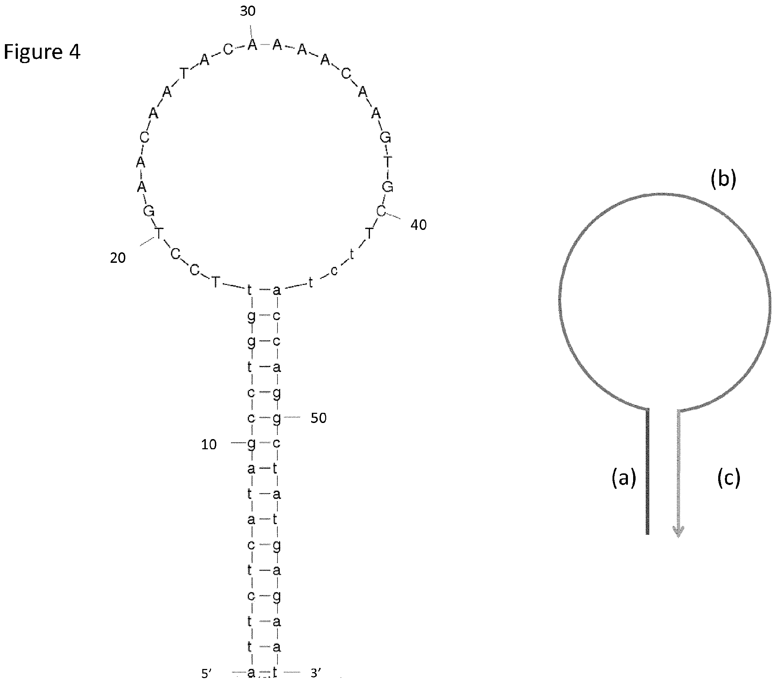

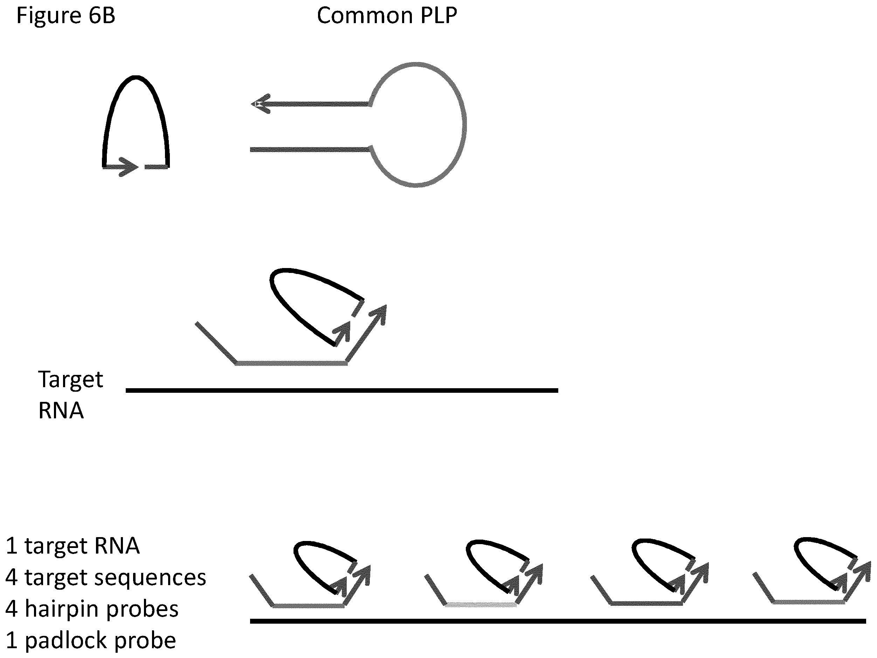

The second domain may lie in the 3' end region of the hairpin probe, which forms the stem duplex of the hairpin probe. In this way, the second domain of the hairpin probe may be released upon binding of the hairpin probe, and made available, or accessible, for binding of the circular or circularisable probe. Such a configuration is depicted in Figures 4 and 6B.

In such a design, and also in the second representative embodiment as shown in Figure 6A, the second domain may be designed to have no sequence homology (i.e. identity) to the target RNA, or complementarity to the first domain, i.e. it is independent of the target RNA or first domain of the probe (in other words, it has no sequence relationship to either the target RNA (i.e. it is orthogonal to it), nor to the first domain). Accordingly, in a probe set of hairpin probes, the second domain of each hairpin probe in the probe set can be the same (i.e. the second domain of each member of the hairpin probe set may be the same). Advantageously, this means that the same circular or circularisable probe can be used for each member of the hairpin probe set. In other words a single circular or circularisable probe may be cognate for all the members of the hairpin probe set. This is also shown in Figure 6A. As discussed below, this means that a common circular or circularisable probe may be used, and that a common signal may be generated. In such an embodiment the common signal is the cumulative signal from all the hairpin/circular or circularisable probes which have bound to different target sequences in the target RNA. In a multiplex embodiment for detection of multiple RNA targets, different hairpin probe sets will be specific for each separate target, and each probe set will have its own, distinguishable cognate circular or circularisable probe.

Alternatively, as discussed further below, the second domain of each member of a hairpin probe set may be different. This will be the case for a probe of the first representative design as shown in Figures 2 and 3, and 5, and may be the case for the second and third representative embodiments as shown in Figures 6A and 6B. Thus, each member of the probe set may have its own, different, cognate circular or circularisable probe (with different regions of complementarity for the second domain), allowing each member to report separately. Thus, each target sequence may be detected separately. In such an embodiment the circular or circularisable probe may be viewed as "unique", giving rise to a signal which is unique to a particular hairpin probe, and hence unique to a particular target sequence.

Advantageously, in the method the circular or circularisable probe is not able to hybridise simultaneously to the hairpin probe and the target RNA. Whilst in certain embodiments the circular or circularisable probe may comprise a region which is complementary to the target RNA, this same region is also complementary to the hairpin probe and the designs of the respective probes, and/or reaction conditions, are such that circular or circularisable probe does not hybridise to the target RNA. Further, the design of the respective probes may be such that a 3-way junction RCA reaction is not possible. In certain other embodiments the circular or circularisable probe is not able to hybridise to the target RNA. In particular it does not contain a region of complementarity to the target RNA.

The term "hybridisation" or "hybridises" as used herein refers to the formation of a duplex between nucleotide sequences which are sufficiently complementary to form duplexes via Watson-Crick base pairing, or any analogous base-pair interactions. Two nucleotide sequences are "complementary" to one another when those molecules share base pair organization homology. Hence, a region of complementarity in a domain of a probe refers to a portion of that domain that is capable of forming an intra- or intermolecular duplex, i.e. either a duplex within the same molecule (a hairpin structure) or a duplex with a different molecule, e.g. the target RNA or a different (cognate) probe. Hybridisation does not require 100% complementarity between the sequences, and hence regions of complementarity to one another do not require the sequences to be fully complementary, although this is not excluded. Thus, the regions of complementarity may contain one or more mismatches. Accordingly, "complementary", as used herein, means "functionally complementary", i.e. a level of complementarity sufficient to mediate a productive hybridisation, which encompasses degrees of complementarity less than 100%. The degree of mismatch tolerated can be controlled by suitable adjustment of the hybridisation conditions. Those skilled in the art of nucleic acid technology can determine duplex stability empirically considering a number of variables including, for example, the length and base pair composition of the respective molecules or probe oligonucleotides, ionic strength, and incidence of mismatched base pairs, following the guidance provided by the art. Thus the design of appropriate probes, and domains thereof, and the conditions under which they hybridise to their respective targets is well within the routine skill of the person skilled in the art.

A region of complementarity, such as for example to a target sequence, in the first domain of the hairpin probe, or to the second domain, at the 3' or 5' end of a circularisable probe, may be at least 6 nucleotides long, to ensure specificity of binding, or more particularly at least 7, 8, 9 or 10 nucleotides long. The upper limit of length of the region is not critical, but may for example be up to 50, 40, 35, 30, 25, 20 or 15 nucleotides. A complementary region may thus have a length in a range between any one of the lower length limits and upper length limits set out above. In the case of a circularisable probe which has 5' and 3' end regions of complementarity to regions in the second domain, the length of an individual complementary end region may be in the lower ranges, so that the total length of the two end regions when hybridised to the second domain is within the upper ranges. For example an individual 5' or 3' end region may be 8-15, e.g. 10-12 nucleotides, so that the total hybridised length is 16-30 nucleotides long, e.g. 20-24. It may be desirable, within the constraints of conformation of the probes, and spacing of the domains, and desired or favoured hybridisations, to minimise the total length or a circular or circularisable probe to minimise the size of the circle which is subjected to RCA, and hence to minimise the lengths of the complementary regions where possible.

As explained above, in the case of a circularisable probe, the total hybridized region which is hybridised to the second domain of the hairpin probe may in some embodiments be made up not only of the target-complementary 3' and 5' end regions of the probe, but may include also either a gap oligonucleotide or other gap-fill sequence (e.g. a gap sequence generated by extension of a 3' end of the probe). In the gap-fill embodiments, the gap may be from 1-20 nucleotides long, e.g. 1-19, 1-18, 1-15, 1-12, 1-10, 1-8, 1-7, 1-6, 1-5, 1-4, or from 2, 3, 4, 5, or 6 to any of the upper limits of the above-noted ranges.

Similarly the lengths of the other regions or domains of the probes may be selected based on the particular nature of the probe, its conformation (e.g. the hairpin structure) and desired or favoured hybridisations etc., e.g. relative stability of the stem duplex of the hairpin probe and such like. The design of the probes may be based on principles well known in the art. The first and second domains of the hairpin probes may be separated by spacer sequences, for example in the loop. The length of the toehold region of a hairpin probe (5' single-stranded region) may vary, depending for example on the target sequence and or probe design, and may be for example be 10-30 nucleotides long, e.g. 12-20.

The lengths of the stem and loop regions of a hairpin probe may be designed or selected based on principles well known in the art for the design of such probes. This may depend also upon where in the hairpin probe the first and second domains are located. Accordingly, purely by way of example, the stem regions may be at least 8, e.g. at least 9 or 10 nucleotides in length, up to for example, 30, 25 or 20 nucleotides in length. The loop may be for example at least 8 nucleotides, e.g. at least 9, 10, 11, 12, 15, 20, 25 or 30 nucleotides.

As discussed above, mismatches may be included in complementary regions, for example to achieve or control the relative strengths of hybridisation between different regions or domains. In particular, the stem of the hairpin structure of the hairpin probe may contain on or more mismatches. In this way, the hairpin may be designed to ensure that it opens upon binding to the target RNA. This will depend on the length of the stem, but may be for example 1-5, e.g. 1-4, 1-3 or 1-2 mismatches.

The circular or circularisable probes, which provide or form the RCA templates, may comprise a reporter domain, which is a region of the probe or sequence that can be used to detect and/or identify the RCA product, i.e. the primer extension product templated by the RCA template. This is particularly advantageous in multiplex embodiments of the invention, where more than one different RNA target is detected in a single assay. It may also be used to distinguish between different target sequences in a target RNA. The reporter domain may be a separate domain, for example included with the "backbone" region of a padlock probe, or other circularisable probe, which lies between the 3' and 5' end regions of the circularisable probe (which are complementary to the second domain), or the duplex region of the dumbbell probe. In other embodiments, the reporter domain may, as noted above, be the region of complementarity in the circular or circularisable probe to the second domain (more specifically to the region in the second domain which is complementary to the target binding site in the first domain). Thus, the reporter domain may comprise, or may overlap with, all or a portion of the region of the circular or circularisable probe which is complementary to the second region of complementarity in the second domain of the hairpin probe.

The reporter domain may thus be a marker or identification sequence in the circular or circularisable probe. In other words, it may be a tag or a bar-code sequence, which allows the probe to be detected and identified. In an embodiment, the reporter domain may allow the circular or circularisable probe, and hence the RCA product it generates, to be distinguished, i.e. from other circular or circularisable probes. As will be clear from the discussion of unique and common circular or circularisable probes above, the reporter domain may be unique for each circular or circularisable probe (including both probes cognate to hairpin probes or probe sets specific for different RNA targets, and probes cognate for the different members of a hairpin probe set), or it may common in the circular or circularisable probes which are cognate for the different members of a hairpin probe set. As discussed above, this means the signal which is generated from the common reporter domain in the circular or circularisable probes is common (i.e. the RCA product will comprise complementary copies of the same reporter domain) and may be detected in a common manner, to provide a cumulative signal arising from all the hairpin probes in a probe set. As also discussed above, in certain embodiments the same circular or circularisable probe may be used for all members of a hairpin probe set, and hence the reporter domain will inevitably be the same for each hairpin probe. However, it may also be the case that different circular or circularisable probes will be cognate for different hairpins, i.e. that each hairpin probe in a set will have its own cognate circular or circularisable probe. This will be the case where the second domain is different in the different members of hairpin probe set, for example in the case of the first representative hairpin embodiment discussed above. In such a case, for a given hairpin probe set, the circular or circularisable probes which are cognate for the different members of the hairpin probe set may each have the same reporter domain, although the binding site for the second domain will be different. In this way, the circular or circularisable probes cognate for a particular hairpin probe set can be common for the hairpin probe set, in the sense that the reporter domain for a particular probe set is common.

In another embodiment, the reporter domain in each cognate circular or circularisable probe for a given hairpin probe set may be different, allowing a different and distinguishable signal to be generated for each hairpin probe in the set. This allows each target sequence in a target RNA to be detected separately. If desired, a combined, cumulative, value for the target RNA may be determined from such individual signals from a hairpin probe set.

The reporter domain in the circular or circularisable probe may comprise the sequence of a specific detection probe (detection oligonucleotide) which is used to detect the RCA product. The RCA product is complementary to the RCA template and as such detection probes that hybridize to the RCA product will comprise a sequence that is identical to part of the RCA template.

Thus, in multiplex assays the RCA template which is used with or generated for the hairpin probe or probe set for each different RNA target may comprise a different reporter domain. Analogously, or additionally, where it is desired to detect the different target sequences within each target RNA separately, the RCA template for each member of the hairpin probe set may comprise a different reporter domain. The detection of the RCA product may be conducted in parallel (i.e. at the same time, for the same sample), e.g. using detection probes tagged with distinct fluorophores that may hybridise to the complement of the reporter domain in the RCA product. Alternatively, each reporter domain (and therefore each target RNA and/or target sequence) may be detected using sequential visualisation reactions, wherein each reaction is separated by, e.g. stripping or bleaching steps. Methods of sequential visualisation reactions suitable for using the methods of the invention are known in the art, e.g. Göransson et al., 2009 (A single molecule array for digital targeted molecular analyses. Nucleic Acids Res. 2009 Jan; 37(1):e7), Wählby et al., 2002 (Sequential immunofluorescence staining and image analysis for detection of large numbers of antigens in individual cell nuclei. Cytometry, 47(1):32-41, 2002). In some representative embodiments of the invention, multiple RNA targets may be detected in parallel. In other representative embodiments of the invention, multiple RNA targets may be detected sequentially.

In certain other embodiments, the reporter domain may not be dependent on sequence. For example it may be a region of duplex structure, to which an intercalating agent may bind, for example as in the case of dumbbell probe. Dumbbell probes may be useful in the detection of miRNAs.

The RCA products may be detected using any convenient protocol, where the particular protocol employed may detect the RCA products non-specifically or specifically, as described in greater detail below. For instance, the RCA product may be detected directly, e.g. the concatemer may be cleaved to generate monomers which may be detected using gel electrophoresis, or more preferably by hybridizing labelled detection oligonucleotides that hybridize to the reporter domain complement in the RCA product. The latter may particularly be used in *in situ* methods. The detection probe need not, however, be directly labelled. For example the detection probe may be an unlabelled probe which functions as a sandwich probe. The concept of sandwich probes is well known in the art and may be applied according to any convenient protocol. The sandwich probes can bind to the RCA product but are not directly labelled themselves; instead, they comprise a sequence to which labelled secondary oligonucleotides can bind, thus forming a "sandwich" between the RCA product and the labelled secondary oligonucleotide. An RCA product may also be detected using non-sequence-specific nucleic acid labelling methods, e.g. DNA binding stains, or by using labelled nucleotides for incorporation into the RCP. Alternatively, the RCA product may be detected indirectly, e.g. the product may be amplified by PCR and the amplification products may be detected.

The analysis of many RNA targets and/or target sequences simultaneously in a single reaction using several different hairpin probes or probe sets (multiplexing) may be enhanced by the increased sensitivity, and in certain embodiments also increased specificity, which may be obtained using the methods and probes of the present invention. Each probe or probe set can be designed to produce an RCA product that can be used to determine the presence or absence, quantity and/or location of the RNA target, or target sequence, ultimately being interrogated. The RCA product may be detected using any of the well-established methods for analysis of nucleic acid molecules known from the literature including liquid chromatography, electrophoresis, mass spectrometry, including CyTOF, microscopy, real-time PCR, fluorescent probes, microarray, colorimetric analysis such as ELISA, flow cytometry, mass spectrometry, or by turbidometric, magnetic, particle counting, electric, surface sensing, weight-based detection techniques. Generally speaking such techniques are relevant for in solution assays.

For a localised *in situ* detection method, the RCA product may generally be detected using labelled detection probes, which may be labelled with any detectable label, which may be directly or indirectly signal-giving. For example the label may be spectroscopically or microscopically detectable, e.g. it may be a fluorescent or colorimetric label, a particle or an enzymatic label. Any of the labels used in immunohistochemical techniques may be used.

In multiplex procedures for detecting different RNA targets and/or target sequences different RCA products arising from different RCA templates (i.e. different circular or circularisable probes, or different reporter domains thereof) may be detected and distinguished by *in situ* sequencing (for example Ke, et al. 2013 Nat Methods), including for example sequencing by synthesis and sequencing by ligation, next generation sequencing and/or sequential and/or combinatorial labelling by hybridisation of labelled detection probes, according to techniques well known in the art. The amount of hairpin probe that is added to a sample may be selected to provide a sufficiently low concentration of hairpin probe in the reaction mixture to minimise non-target specific interactions, i.e. to ensure that the hairpin probe will not randomly bind to non-target molecules in the sample to any great or substantial degree. For example, it is intended that only when the hairpin probe binds its target RNA is the primer sequence released and the RCA product generated. In representative embodiments, the concentration of each hairpin probe in the reaction mixture following combination with the sample ranges from about 1 fM to 1µM, such as from about 1pM to about 500 nM, e.g. 1, 2, 5, 10, 20, 50, 60, 70, 80, 90 or 100 nM. The hairpins may thus be used such that the target RNA is present in excess. However, in other embodiments the hairpin probes may be used in excess of the target RNA.

A number of different hairpin probes, or probe sets, may be added to a sample for a multiplex assay in order to detect multiple RNA targets in parallel in the same reaction mixture. Multiplex assays may involve the detection of tens, hundreds, thousands or even tens of thousands of RNA sequences in a sample. Accordingly, multiplex assays may comprise at least 2 distinct hairpin probes, i.e. probes capable of hybridising to different RNA targets or target sequences. For instance, multiplex assays may utilise at least 3, 4, 5, 10, 20, 30, 40 or 50 probes, such as 100, 200, 500, 1000 or more probes.

Following combination of the sample and the hairpin probe(s), the reaction mixture may be incubated for a period of time sufficient for the hairpin probe(s) to bind to its target, if present, in the sample. Similarly, once the circular or circularisable probe(s) have been added to the sample, the reaction mixture may be incubated to allow the circular or circularisable probe(s) to bind to the unfolded hairpin probes. As described above, once a circularisable probe has bound to the unfolded hairpin probe, it is ligated to generate the RCA template. The second domain of the hairpin probe acts as a ligation template.

As is known in the art, in template-directed ligation ligases catalyze the formation of a phosphodiester bond between juxtaposed 3'-hydroxyl and 5'-phosphate termini of two immediately adjacent nucleic acids when they are annealed or hybridized to a third nucleic acid sequence to which they are complementary (i.e. a ligation template). Any convenient ligase may be employed, where representative ligases of interest include, but are not limited to: Temperature sensitive and thermostable ligases. Temperature sensitive ligases include, but are not limited to, bacteriophage T4 DNA ligase, bacteriophage T7 ligase, and *E. coli* ligase. Thermostable ligases include, but are not limited to, Taq ligase, Tth ligase, Ampligase^{®} and Pfu ligase. Thermostable ligase may be obtained from thermophilic or hyperthermophilic organisms, including but not limited to, prokaryotic, eukaryotic, or archael organisms. Certain RNA ligases may also be employed in the methods of the invention.

A suitable ligase and any reagents that are necessary and/or desirable may be combined with the reaction mixture and maintained under conditions sufficient for ligation of the hybridized oligonucleotides to occur. Ligation reaction conditions are well known to those of skill in the art. During ligation, the reaction mixture in certain embodiments may be maintained at a temperature ranging from about 4°C to about 105°C, about 4 to about 80°C, such as about 10 to about 70°C, about 15 to about 60°C, typically such as from about 20°C to about 37°C for a period of time ranging from about 5 seconds to about 16 hours, such as from about 1 minute to about 1 hour. In yet other embodiments, the reaction mixture may be maintained at a temperature ranging from about 35°C to about 45°C, such as from about 37°C to about 42°C, e.g., at or about 38°C, 39°C, 40°C or 41°C, for a period of time ranging from about 5 seconds to about 16 hours, such as from about 1 minute to about 1 hour, including from about 2 minutes to about 8 hours.

It will be evident that the ligation conditions may depend on the ligase enzyme used in the methods of the invention. Hence, the above-described ligation conditions are merely a representative example and the parameters may be varied according to well known protocols.

The next step of the method following the ligation step is to generate the RCA product. The RCA reaction is primed by the second domain of the hairpin probe. Thus the second domain may lie at the 3' end of the hairpin probe and, once the hairpin has been opened it may be hybridised to the circular or circularised probe in such a manner that it can prime the RCA. However, the extensible 3' end of the second domain may be generated by exonuclease digestion of the 3' end of the hairpin probe (see below).

Procedures for carrying out RCA reactions are well known in the art. In addition to the above nucleic acid components, the RCA reaction mixture includes a polymerase, e.g. phi29 DNA polymerase and other components required for a DNA polymerase reaction as described below. The desired polymerase activity may be provided by one or more distinct polymerase enzymes. In some embodiment the polymerase has exonuclease activity, e.g. 5' and/or 3' exonuclease activity. It may be desirable to use a polymerase having 3' exonuclease activity, such as phi29 or phi29-based polymerases, as this exonuclease activity may, if necessary, digest the 3' end of the hairpin probe to generate a hybridised 3' end which can act as a primer for the RCA reaction.

In preparing the reaction mixture of this step of the subject methods, the various constituent components may be combined in any convenient order. For example, all of the various constituent components may be combined at the same time to produce the reaction mixture.

The amplified products of the RCA reaction may be detected using any convenient protocol, as discussed above. Representative non-specific detection protocols of interest include protocols that employ signal producing systems that selectively detect single or double stranded DNA products, e.g., via intercalation. Representative detectable molecules that find use in such embodiments include fluorescent nucleic acid stains, such as phenanthridinium dyes, including monomers or homo- or heterodimers thereof, that give an enhanced fluorescence when complexed with nucleic acids. Examples of phenanthridinium dyes include ethidium homodimer, ethidium bromide, propidium iodide, and other alkylsubstituted phenanthridinium dyes. In another embodiment the nucleic acid stain is or incorporates an acridine dye, or a homo- or heterodimer thereof, such as acridine orange, acridine homodimer, ethidium-acridine heterodimer, or 9-amino-6-chloro-2-methoxyacridine. In yet another embodiment, the nucleic acid stain is an indole or imidazole dye, such as Hoechst 33258, Hoechst 33342, Hoechst 34580 (BIOPROBES 34, Molecular Probes, Inc. Eugene, Oreg., (May 2000)) DAPI (4',6-diamidino-2-phenylindole) or DIPI (4',6-(diimidazolin-2-yl)-2-phenylindole). Other permitted nucleic acid stains include, but are not limited to, 7-aminoactinomycin D, hydroxystilbamidine, LDS 751, selected psoralens (furocoumarins), styryl dyes, metal complexes such as ruthenium complexes, and transition metal complexes (incorporating Tb³⁺ and Eu³⁺, for example). In certain embodiments, the nucleic acid stain is a cyanine dye or a homo- or heterodimer of a cyanine dye that gives an enhanced fluorescence when associated with nucleic acids. Any of the dyes described in U.S. Pat.No.4,883,867 to Lee (1989), U.S. Pat. No. 5,582,977 toYue et al. (1996), U.S. Pat. No. 5,321,130 to Yue et al. (1994), and U.S. Pat. No. 5,410,030 to Yue et al. (1995) may be used, including nucleic acid stains commercially available under the trademarks TOTO, BOBO, POPO, YOYO, TO-PRO, BO-PRO, PO-PRO and YO-PRO from Molecular Probes, Inc., Eugene, Oreg. Any of the dyes described in U.S. Pat. No. 5,436,134 to Haugland et al. (1995), U.S. Pat. No. 5,658,751 to Yue et al. (1997), and U.S. Pat. No. 5,863,753 to Haugland et al. (1999) may be used, including nucleic acid stains commercially available under the trademarks SYBR Green, EvaGreen, SYTO, SYTOX, PICOGREEN, OLIGREEN, and RIBOGREEN from Molecular Probes, Inc., Eugene, Oreg. In yet other embodiments, the nucleic acid stain is a monomeric, homodimeric or heterodimeric cyanine dye that incorporates an aza- or polyazabenzazolium heterocycle, such as an azabenzoxazole, azabenzimidazole, or azabenzothiazole, that gives an enhanced fluorescence when associated with nucleic acids, including nucleic acid stains commercially available under the trademarks SYTO, SYTOX, JOJO, JO-PRO, LOLO, LO-PRO from Molecular Probes, Inc., Eugene, Oreg.

In yet other embodiments, a signal producing system that is specific for the RCA product, as opposed to nucleic acid molecules in general, may be employed to detect the RCA product. In these embodiments, the signal producing system may include a detection probe nucleic acid or oligonucleotide (a detection probe) that specifically binds to a sequence found in the RCA product (i.e. a reporter domain sequence), where the detection probe may be labelled with a directly or indirectly detectable label. A directly detectable label is one that can be directly detected without the use of additional reagents, while an indirectly detectable label is one that is detectable by employing one or more additional reagents, e.g., where the label is a member of a signal producing system made up of two or more components. In many embodiments, the label is a directly detectable label, where directly detectable labels of interest include, but are not limited to: fluorescent labels, radioisotopic labels, chemiluminescent labels, and the like. In many embodiments, the label is a fluorescent label, where the labelling reagent employed in such embodiments is a fluorescently tagged nucleotide(s), e.g. fluorescently tagged CTP (such as Cy3-CTP, Cy5-CTP) etc. Fluorescent moieties which may be used to tag nucleotides for producing labelled probe nucleic acids (i.e. detection probes) include, but are not limited to: fluorescein, the cyanine dyes, such as Cy3, Cy5, Alexa 555, Bodipy 630/650, and the like. Other labels, such as those described above, may also be employed as are known in the art.

Products of the present invention and disclosure include hairpin probes, probe sets and kits as defined and discussed above. In addition to the components set out above, the kits may also contain one or more other component such as reagents to perform one or more of the steps of the methods. Such components may include a ligase, a polymerase for the RCA reaction (and/or for a gap-filling extension reaction if appropriate) and means or reagents for detecting the RCA product, including for example detection probes.

The kit may further optionally include nucleotides and/or buffers and/or reaction solutions for performing one or more of the steps of the method. The kit may further include instructions for performing the method. These may be for example in printed form, or on a computer-readable medium, or as a website address.

Advantages of the methods and probes disclosed and described herein are discussed above. Such advantages are particularly beneficial in the context of localised in situ detection.

Advantages include that the streamlined design and preparation of probes for the detection of RNA is enabled. The target-specific probes which bind to the target RNA (hairpin probes) do not need to be labelled. In view of the signal amplification afforded by the method, the number of hairpin probes required, as compared to smFISH, is reduced. The method further allows the ability to detect mRNA, and hence to image gene expression, over a wide portion of a tissue section at low magnification (e.g. X20 objective) and is applicable to archival FFPE tissue sections and aged FFPE samples in which smFISH and other fluorescence-based RNA detection strategies are notoriously challenging. The method may also have applicability to tissue microarrays for high throughput screening of potentially useful biomarkers. By using hairpin probes, rather than linear target-specific probes, less background and non-specific signals may be generated, improving the specificity of the method.

Such features make the method a powerful tool that can be integrated in routine pathological diagnostics, i.e. the method is suited to use in clinical diagnostic laboratories. Furthermore, it may also be a useful research tool, for example to study or quantify expression levels and investigate the spatial distribution of gene expression. This may be of particular use in neuroscience, to chart the spatial morphology of genes in brain samples, or to identify cellular sub-types on the basis of gene expression patterns. The method can be used to identify the spatial location of both lowly expressed and newly predicted cellular subtypes based on the detection of a set of transcripts that specifically marks a given cell subtype due to its specificity. High-throughput spatial transcriptomic techniques, including sequential smFISH, spatial transcriptomics and MERFISH, have also been applied to map cellular diversity in human and mouse brain. However, these methods are technically challenging and still require posterior validation by lower-throughput assays such as smFISH. In this context, the present method could prove very valuable especially when samples characterized by high levels of tissue autofluorescence need to be analyzed (e.g., aged brain).

Finally, as the method allows the detection of a strong signal already with 4-5 hairpin probes, the method could be applied to detect short RNAs or to discriminate between different splicing variants, which is not possible by smFISH. In summary, the method is a versatile, scalable and cost-effective method that can be used to quantify individual RNA molecules also in challenging FFPE samples, with broad applications ranging from research to routine diagnostics.

Although described herein with reference to circular or circularisable probes and RCA, the principles of the present method may be more widely applicable, and the method may be adapted for use with other amplification procedures, wherein rather than harbouring a primer for an RCA reaction, the second domain of the hairpin probe may instead contain another component for a different amplification reaction. Thus, in an analogous manner to that described above, the hairpin probe may be opened upon binding to its target sequence to release such component and make it accessible, or available, to bind to a cognate amplification reaction component, in a manner that initiates an amplification reaction, or allows it to take place.

Accordingly, in another, broader, aspect, the present disclosure can be seen to provide a method for detection of target RNA in a sample comprising:
(a) contacting said sample with at least one target-specific hairpin probe comprising;
   (i) a first domain which comprises a first region of complementarity to a target sequence in said target RNA and is capable of hybridising to said target RNA;
   (ii) a second domain which is not capable of hybridising to the target RNA, which comprises a second region of complementarity to a cognate amplification reaction component, and which is capable of acting as an activator to initiate an amplification reaction;
   wherein the second domain is at least partly comprised within a hairpin structure of the probe, such that the second domain is not capable of binding to said cognate amplification reaction component and/or initiating an amplification reaction until released from said hairpin structure, and allowing said hairpin probe to hybridise to the target RNA, wherein hybridization of said first domain to said target sequence causes the hairpin structure to open and release said second domain;
(b) contacting said sample with an amplification reaction component cognate for said hairpin probe, wherein the amplification reaction component comprises one or more regions complementary to the second region of complementarity in the second domain, and hybridising the amplification reaction component to the hairpin probe at said second domain;
(c) performing an amplification reaction using the amplification reaction component of (b); and
(e) detecting the product of said amplification reaction.

The term "activator" is used broadly herein to include any component of an amplification reaction. In particular, the activator may be a component which initiates an amplification reaction, or which is required for the amplification reaction to take place. The activator may interact with, e.g. hybridise to, another amplification reaction component, to allow an amplification reaction to take place.

For example, the second domain, or a part or region thereof, may be capable of acting as a primer for an amplification reaction, including a polymerase-based amplification reaction. Such a primer may hybridise to an amplification template, analogously to the circular or circularised probe as discussed above. The cognate amplification reaction component may thus be a template molecule comprising at least one region of complementarity to the second region of complementarity in the second domain of the hairpin probe.

In another embodiment, the second domain may be capable of acting as, or may comprise, an initiator for a hybridisation chain reaction. The cognate amplification reaction component may be a HCR monomer capable of hybridising to said initiator. Accordingly, the HCR monomer may comprise a region of complementarity to the second region of complementarity in the second domain of the hairpin probe. More particularly, the toehold region of a first HCR monomer may contain a region which is complementary to the second region of complementarity, or a part thereof.

The HCR reaction is well known and described in the literature, including for example in Dirks and Pierce, 2004, PNAS, 101(43), 15275-15278 and in US 7,632,641 and US 7,721,721 (see also US 2006/00234261, US 2018/0066303; Chemeris et al, 2008 Doklady Biochemistry and Biophysics, 419, 53-55; Niu *et al,* 2010, 46, 3089-3091; Choi et al, 2010, Nat. Biotechnol. 28(11), 1208-1212; and Song et al, 2012, Analyst, 137, 1396-1401.

The amplification reaction of step (c) may thus be performed in a manner known in the art, and may include adding one or more other reaction components for an amplification reaction. For example in the case of HCR, the reaction will require, in addition to the initiator provided by the second domain of the hairpin probe, and a first HCR monomer as the cognate amplification reaction component, a second HCR monomer, which reacts with the first HCR monomer to assemble the HCR polymeric reaction product. Other amplification reactions may require other components, for example a second, and optionally further, amplification primers, or other oligonucleotide components etc.

The hairpin probes and reaction methods may be as described above.

Such other amplification products may be detected according to techniques and principles well known in the art, analogously to the description above for RCA products, which may also apply here. Thus for example, the HCR monomers may contain reporter domains, as described above, and detection probes capable of hybridising to the reporter domains within the HCR product may be used, or the HCR monomers may be labelled etc.

This broader aspect of the disclosure and invention also provides a hairpin probe for use in detecting a target RNA molecule, said probe comprising:
(i) a first domain which comprises a first region of complementarity to a target sequence in a target RNA and is capable of hybridising to said target RNA;
(ii) a second domain which is not capable of hybridising to the target RNA, which comprises a second region of complementarity to a cognate amplification reaction component, and which is capable of acting as an activator for an amplification reaction;
wherein the second domain is at least partly comprised within a hairpin structure of the probe, such that the second domain is not capable of binding to said cognate amplification reaction component and/or initiating an amplification reaction until released from said hairpin structure, and wherein the hairpin structure is such that hybridisation of said first domain to said target sequence causes the hairpin structure to open and release said second domain.

A probe set may comprise two or more target-specific hairpin probes as defined above each probe being specific for a different target sequence in the same target RNA, wherein the first domain of each hairpin probe in the set comprises a region of complementarity to a different target sequence in the target RNA.

A kit may comprise:
(i) a target-specific hairpin probe, or a probe set as defined above; and
(ii) amplification reaction component cognate for said hairpin probe, or amplification reaction components cognate for the hairpin probes in the probe set, each amplification reaction component comprising one or more regions complementary to the second region of complementarity in the second domain of said hairpin probe(s).

Such a kit may comprise (i) a set of amplification reaction components, wherein each member of the amplification reaction component probe set is cognate for a different hairpin probe, or (ii) one or more amplification reaction components wherein each component is cognate for all members of a given hairpin probe set.

In particular embodiments of these aspects the second domain may function as, or may comprise, a HCR initiator, and the cognate reaction component may be a HCR monomer.

The method will now be described in more detail with reference to the following nonlimiting examples.

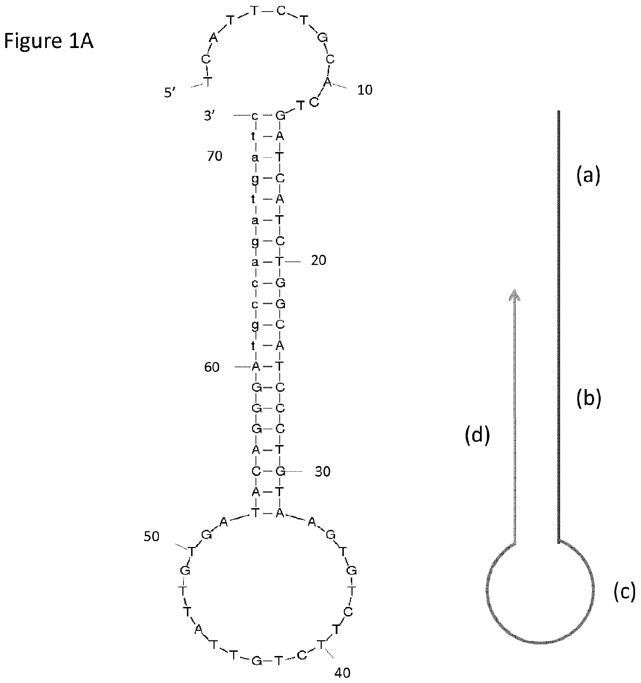

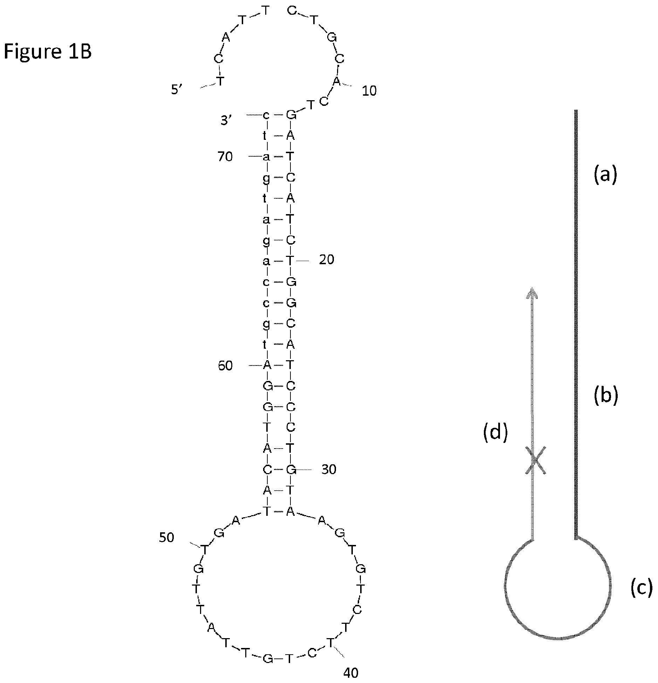

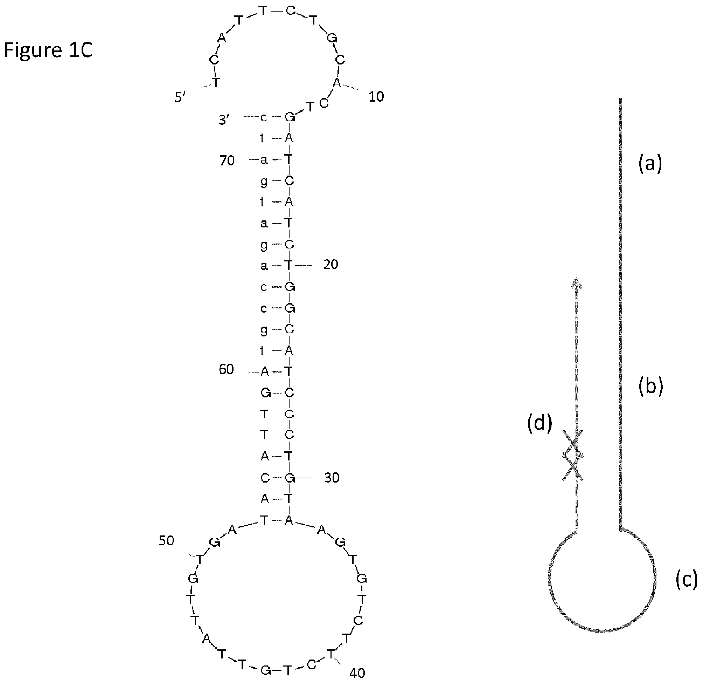

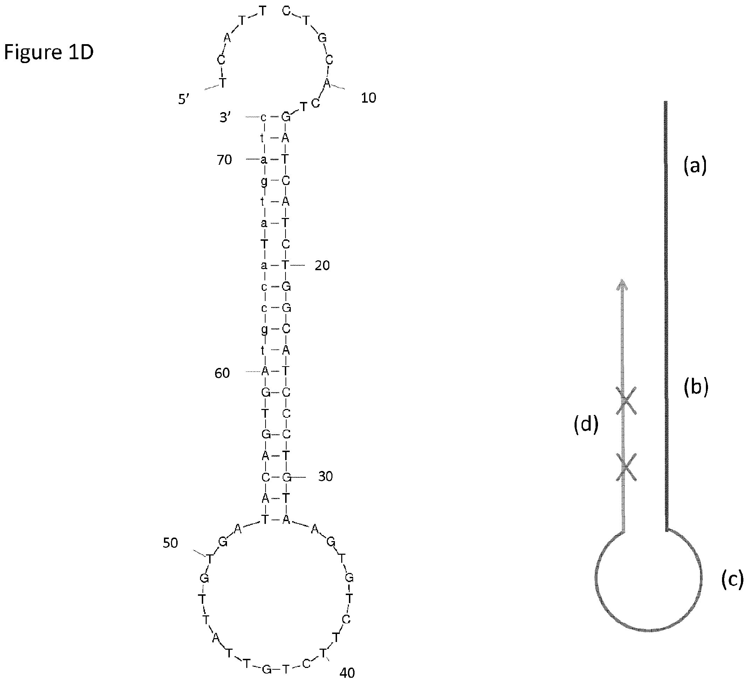

Figure 1 shows the structures of four hairpin probes (Mismatched Hairpin Probes), each 72 nucleotides in length, comprising a 12 nucleotide toehold at the 5' end, a 20 nucleotide stem region and a loop region. The probes also contain varying numbers of mismatches in the stem region.
   (A) shows a probe with no mismatch (SEQ ID NO: 1), wherein (a) is the 5' end, 12nt toehold, (b) is the 20nt stem, with 0 mismatches, (c) is the 20nt loop containing 6nt spacers & 10nt of PLP ligation site, and (d) is the continued PLP ligation site and 3' end. dG = -22.19.
   (B) shows a probe with 1 mismatch at the 5th nucleotide from the loop (SEQ ID NO: 2), wherein (a) is the 5' end, 12nt toehold, (b) is the 20nt stem, with 1 mismatch on the 16^{th} nt of the 20nt stem, (c) is the 20nt loop containing 6nt spacers & 10nt of PLP ligation site, and (d) is the continued PLP ligation site and 3' end, with X marking the mismatch. dG = - 17.72.
   (C) shows a probe with 2 mismatches adjacent to each other at the 5th and 6th nucleotides from the loop (SEQ ID NO: 3), wherein (a) is the 5' end, 12nt toehold, (b) is the 20nt stem, with 2 mismatches adjacent to each other on the 15^{th} and 16^{th} nts of the 20nt stem, (c) is the 20nt loop containing 6nt spacers & 10nt of PLP ligation site, and (d) is the continued PLP ligation site and 3' end, with X marking the mismatches. dG = -15.23.
   (D) shows a probe with 2 mismatches spaced at the 6th and 14th nucleotides from the loop (SEQ ID NO: 4), wherein (a) is the 5' end, 12nt toehold, (b) is the 20nt stem, with 2 mismatches spaced apart from each other on the 7^{th} and 15^{th} nts of the 20nt stem, (c) is the 20nt loop containing 6nt spacers & 10nt of PLP ligation site, and (d) is the continued PLP ligation site and 3' end, with X marking the mismatches. dG = -12.63.
Figure 2 shows the general structure of an alternative probe design (Non-mismatched Hairpin Probes).
   (A) shows a probe comprising a 12 nucleotide toehold at the 5' end, and a similar 20 nucleotide stem region and a loop region (SEQ ID NO: 5), wherein (a) is the 5' end, 12nt toehold, (b) is the 20nt stem, (c) is the 20nt loop containing 3nt spacers & 10nt of PLP ligation site, and (d) is the continued PLP ligation site and 3' end. dG = -20.38.
   (B) shows a similar probe with a longer, 20 nucleotide toehold (SEQ ID NO: 6), wherein (a) is the 5' end, 20nt toehold, (b) is the 20nt stem, (c) is the 20nt loop containing 3nt spacers & 10nt of PLP ligation site, and (d) is the continued PLP ligation site and 3' end. dG = -20.38.
Figure 3 shows the general structure of another alternative probe design comprising a shorter stem (Short Hairpin Probes). These probes similarly comprise a toehold at the 5' end, a stem and a loop region. The probe of (A) has a 12 nucleotide toehold (SEQ ID NO: 7), wherein (a) is the 5' end, 12nt toehold, (b) is the 10nt stem with >60% G-C content, (c) is the loop region containing a 3nt spacer, higher A-T content & PLP ligation site, and (d) is the continued PLP ligation site and 3' end. dG = -11.09.
   The probe of (B) has a 20 nucleotide toehold (SEQ ID NO: 8), wherein (a) is the 5' end, 20nt toehold, (b) is the 10nt stem with >60% G-C content, (c) is the loop region containing a 3nt spacer, higher A-T content & PLP ligation site, and (d) is the continued PLP ligation site and 3' end. dG = -11.09.
Figure 4 shows the general structure of a fourth alternative probe design (Loop Target Hairpin Probe) comprising a stem that starts at the 5' end and ends at the 3' end, with no toehold (SEQ ID NO: 9), wherein (a) is the 5' end, 16nt stem, (b) is the 24nt long loop with a 3nt spacer, and (c) is the 16 nt PLP ligation site and 3' end. The loop region of this probe is made up of the complimentary sequence to the RNA target. dG = -14.29.

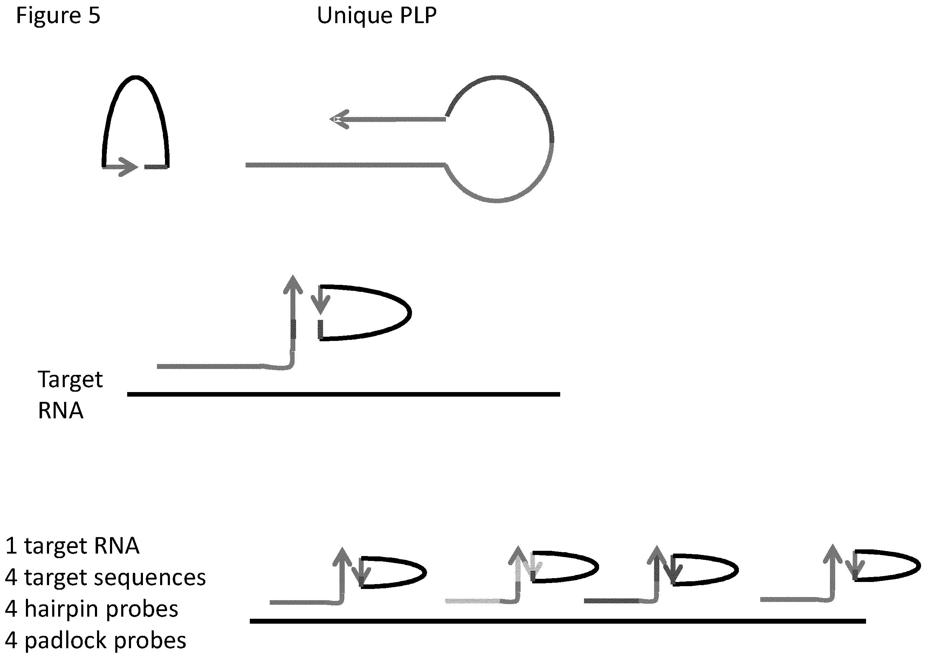

Figure 5 shows a scheme according to one illustrative embodiment wherein the method involves the use of "unique" padlock probes. In this scheme the padlock probe binding site of the hairpin probe comprises part of the loop region and part of the stem. Accordingly, a portion of the padlock probe binding site is complementary to the sequence which hybridizes to the target RNA, and thus the padlock probe must be different for each target sequence. In the example, one target RNA comprising 4 target sequences is bound by 4 hairpin probes, each of which is in turn bound by a cognate padlock probe.
Figure 6 shows alternative illustrative embodiments involving the use of "common" padlock probes. In the scheme in (A) the padlock probe binding site of the hairpin probe is entirely within the loop region and thus is unaffected by the sequence of the target RNA. Similarly, in the scheme in (B) the padlock binding site is entirely in the stem and the RNA targeting sequence is entirely in the loop region. Accordingly, in these examples, one target RNA comprising 4 target sequences is bound by 4 hairpin probes, each of which is in turn bound by a copy of the same padlock probe.

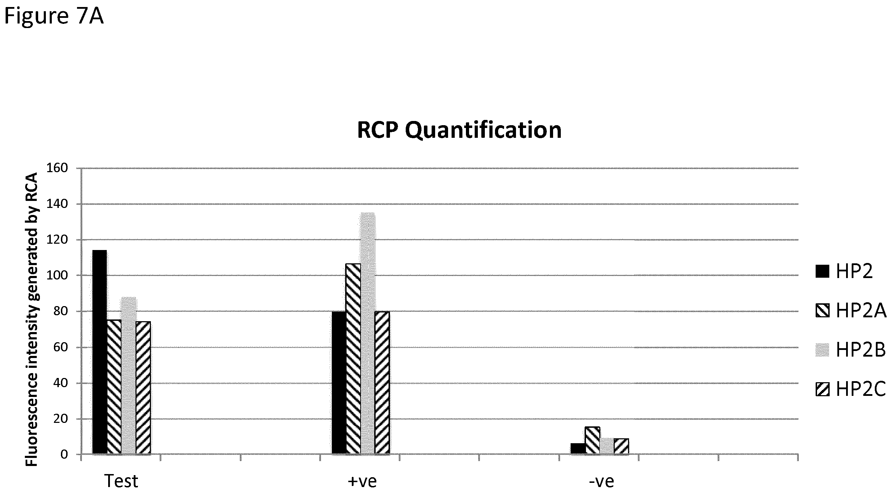

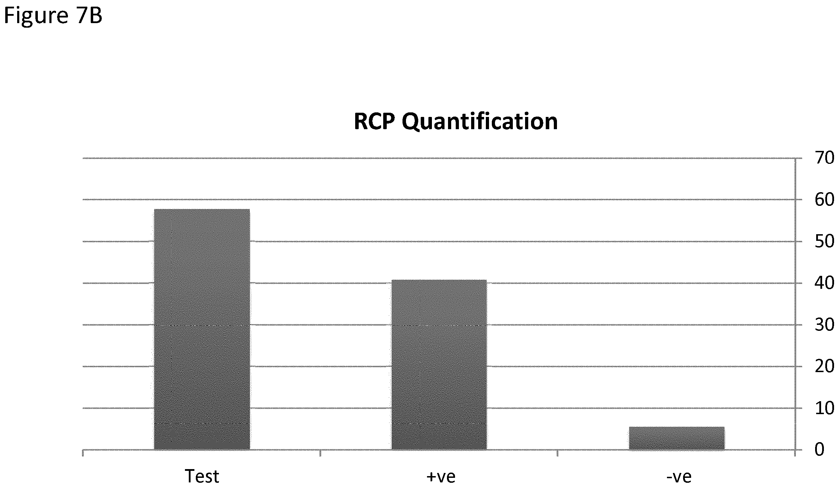

Figure 7 shows the results of an in vitro experiment to confirm that in the presence of a target sequence, hairpin probes can successfully unfold to expose a padlock probe binding site, and thus lead to RCA initiation. Hairpin probes were allowed to hybridize with their mRNA target sequences in solution before padlock probes were added, together with a ligation mix comprising TTH ligase. The samples were then added to an amplification mix comprising Phi29 polymerase and dNTPs and incubated for 1 hour to allow amplification to occur. The resulting RCPs were then visualised using a fluorometer. The experiment was repeated; (i) with the template target sequences in excess of the hairpin probes (Test); (ii) with the hairpin probes in excess of the template target sequences (+ve); and (iii) with no template sequences at all (-ve). The results in figure 7A are from an experiment that was run with 4 different Mismatched Hairpin Probes; HP2, HP2A; HP2B; and HP2C, which correspond to the probes shown in figures 1A-D. The results in figure 7B are from an experiment that was run with the third alternative probe design (Short Hairpin Probe) as depicted in figures 3A and 3B.

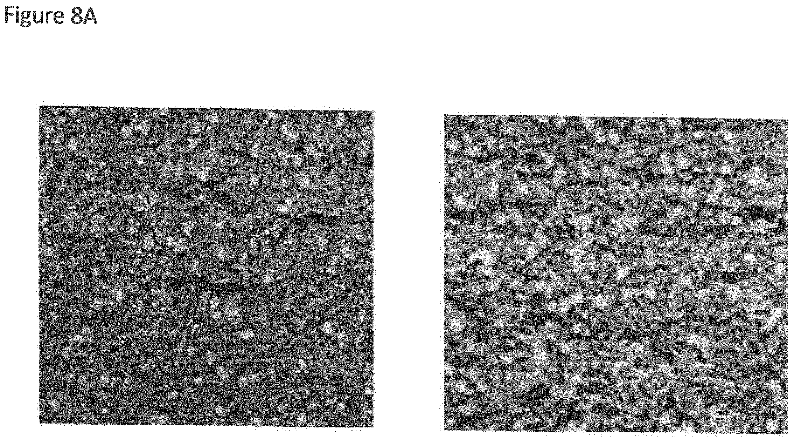

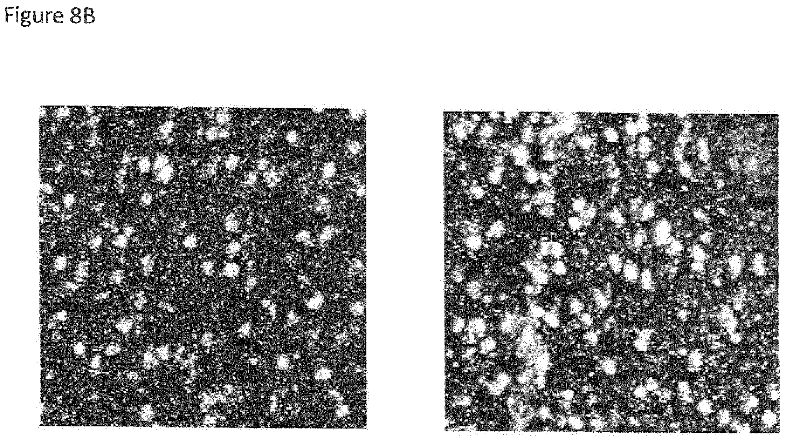

Figure 8 shows the results of an in situ experiment conducted on sections of mouse brain using hairpin probes of the invention and an alternative probe design as a control. The control probes used were linear and comprised a region of complementarity to the target and a 3' end that is not complementary to the target but contains a padlock probe binding site. The samples were first fixed and washed, before hairpin or control probes were added and allowed to hybridise. Padlock probes and ligase were then added, followed by an amplification mix comprising Phi29 polymerase and dNTPs. Finally, detection oligonucleotides were added and the RCPs were detected via fluorescence imaging. The images in (A) were produced using the hairpin probes of the invention, and show the results from a region of tissue expected to highly express the target gene (left) and a region expected to have low levels of expression (right). The images produced using the control probes are shown in (B), again with a region of high predicted expression on the left, and a region of low predicted expression on the right.

### Example 1 - Probe Design & synthesis

### Mismatches in Stem Design

Telomere 72-nucleotide (nt) probes from Integrated DNA Technology (IDT) were designed with a 12-nt toehold, with a 20nt long stem, and with different number of mismatches in the stem:
1. No mismatches
2. 1 Mismatch, 5^{th} nt from loop
3. 2 Mismatches, adjacent 5^{th} and 6^{th} nt from loop
4. 2 Mismatches, spaced, 7^{th} nt and 15^{th} nt from loop

These probes are shown in Figures 1A - 1D, respectively.

The padlock probe binding site was designed to be 30nt in length, with a 6nt spacer in between the binding site and the stem.

The mismatches in the stem of the hairpin probes were designed to destabilize the structure and allow the stem to 'open up' with relative ease in the presence of its target sequence.

### Shorter Stem Design

In addition, Telomere 47-nucleotide (nt) (short toehold) / 55-nt (long toehold) probes from Integrated DNA Technology (IDT) were designed with a 12-nt (short toehold) / 20-nt (long toehold) targeting sequence at the 5' end, a 22-nt sequence for Padlock Probe hybridization and ligation, and a 3-nt spacer in between.

The stems of the hairpin probes were designed to be 10-nt long, with at least 60% G-C content. The loops of the hairpin probes were also designed to contain more A-T than G-C content. The toeholds of hairpin probes designed were 12-nt (short toehold) and 20-nt (long toehold). The structures of these probes are shown in Figure 3.

### Example 2 - In solution methods

As a proof of concept, in order to show that the hairpin probes can successfully unfold upon binding to a target sequence and expose a padlock probe binding site to trigger RCA initiation, an in vitro assay was conducted.

The NeuroD6 gene found in mouse brain was used to design the hairpin probes and padlock probes. In particular, 5 different, non-overlapping sequences of the NeuroD6 mRNA sequence were selected. The hairpin probes were designed to target the 1 site of the NeuroD6 gene. The corresponding sequence of the aforementioned mRNA was ordered from IDT and subsequently tested in solution.

To quantify if designed hairpin probes would hybridize to the target sequence and fully open up with an 'L' overhang for padlock probe binding for amplification, two different test conditions were assessed. Firstly, the reaction was carried out with the hairpin probes in excess, relative to the templates; and secondly, the reaction was repeated with the templates in excess relative to the hairpin probes.

In addition, a negative control consisting of a mixture of hairpin probes and padlock probes only was left to hybridize in the absence of a target sequence, to assess the stability and specificity of the hairpin probes.

### Step 1: Hybridization

Designed hairpin probes were first left to hybridize to the target sequence of the mRNA target of the NeuroD6 gene in 5X SSC, 0.01% Tween Hybridization buffer (5X SSCT).

### Positive Tests:

1. Hairpin probes in excess of templates
   400nM of hairpin probes, 100nM of templates were left to hybridize for 3h at 37C in 2.5X SSC, 0.005 Tween buffer.
2. Templates in excess of hairpin probes
   400nM of templates, 100nM of hairpin probes were left to hybridize for 3h at 37C in 2.5X SSC, 0.005 Tween buffer.

### Negative control:

1. 100nM of hairpin probes were left to incubate for 3h at 37C in 2.5X SSC, 0.005 Tween buffer.

### Step 2: Ligation

Reaction mix from 'Step 1: Hybridization' was then added to Ligation Mix for a 10x dilution of Hybridization Mix. Ligation Mix consists of 1x TTH Buffer, 0.05M KCI, 20% Formamide, 0.2µg/µl BSA, 10.0nM of padlock probes and 0.25u/µl of TTH Ligase. Samples were then incubated at 45C for 30mins.

### Step 3: Amplification & Quantification

Reaction mix from 'Step 2: Ligation' was then added to Amplification Mix for a 10x dilution of Ligation Mix. The Amplification Mix consists of 1x Phi29 Buffer, 0.25mM dNTPs, 0.2µg/µl BSA, 5% Glycerol, 0.2u/µl Phi29 polymerase enzyme. Samples were then incubated at 37C for 1h.

Quantification of RCPs from the amplification step was carried out using a Qubit fluorometer. 5.0 µl of Amplification mix was mixed with 195.0 µl of Qubit working solution which containing a fluorophores that binds to DNA for florescent quantification.

The positive and negative control tests were performed in parallel to validate that signals produced from the positive tests are not attributed to padlock probes 'opening' the hairpin probes to undergo binding, ligation and subsequently RCA. This validates that hairpin probes will remain hybridized to themselves in the absence of the target template and not open up for padlock probe binding, thus avoiding RCA initiation.

The results obtained from the positive tests validated that signals can be attributed to the fact that the hairpin probes 'opened up' to hybridize to the templates, exposing the padlock probe binding site for the padlock probe to bind, circularize and subsequently undergo RCA, producing RCPs.

The results from these experiments are shown in Figure 7.

### Example 3 - In Situ Methods

In situ experiments were conducted on 10µm sections of mouse brain of C57BL/6J mice. In addition to the hairpin probes of the present invention, this experiment also used an alternative probe design as a control. The control system involved the use of linear (non-hairpin) probes comprising a region of complementarity to the target and a 3' end that is not complementary to the target but contains a padlock probe binding site. As with the present hairpin probes, the padlock probe can bind, become circularized via ligation and initiate RCA. These linear probes (control probes or control primers), however, can be targeted by padlock probes and initiate RCA even in the absence of the target template, hence generating unspecific background signals in vitro and in tissue sections where linear probes bind and/or stick to the tissue matrix unspecifically.

### Step 1: Fixation

All samples were first fixed and incubated in a fixation solution containing 3.7% PFA in DEPC-PBS solution in room temperature for 5mins. The samples were then incubated in Wash Buffer 1 (WB1) solution containing DEPC-PBS for 1 min, before incubating at RT for 5 mins in Permeabilization Solution (PS), containing (0.1M HCI in DEPC H₂O). Next, the samples were then washed in WB1 twice, before incubating in 70% and 100% EtOH for 2mins respectively. Lastly, a 50ul SecureSeal Chamber (Grace Bio-Labs) was then mounted onto the slide to facilitate hybridization in Step 2.

### Step 2: Probe Hybridization

Wash Buffer 2 (WB2) containing DEPC-PBS-Tween 0.05% was applied to the SecureSeal Chambers, whilst a Probe Mix containing 2X SSC, 10% Formamide, 10mM MgCl₂ and 50nM hairpin or control probes was prepared. The Probe Mix was then added to the chambers and left to incubate for 3h at 37C.

### Step 3: Probe Ligation

Samples were then washed thrice in WB2 before Ligation Mix containing 1x TTH Buffer, 0.2µg/µl BSA, 0.05M KCI, 100nM padlock probes, 20% Formamide and 0.5µg/µl TTH Ligase was added. The slides were then incubated at 37C for 1h.

### Step 4: Amplification & Florescent Labeling

Samples were washed twice in WB2, before Amplification Mix containing 1x Phi 29 Buffer, 0.25mM dNTP, 0.2µg/µl BSA, 10% glycerol and 1µg/µl Phi29 Polymerase enzyme was added. The samples were then incubated either at 30C overnight, or at 37C for 3h. The Amplification Mix was then removed from the chambers, which were washed with WB2 thrice, leaving the last wash buffer in the chamber. The samples were then incubated in 2x SSC, 20% formamide and 100nM of detection oligos for 30 minutes in darkness at room temperature. Unbound detection oligos were then removed with three washes with WB2.

The quantification blobs from experiments were validated by imaging and running images through CellProfiler pipelines. Images were taken under Nikon Ti2 microscope, running on NIS Elements AR software. The images for gene expression in selected area was selected to be 2x 2x2 tile region in the highly and lowly expressed region of the samples, based on publicly available data from the Allen Cell Atlas. The selected areas of interests were then imaged with 20X objective with illumination of Cy3 and DAPI. A stack of images at different focal depths were imaged for RCPs located in different focal Z planes, and were then merged to a single image using the 'Create Maximum Intensity Projection (MIP) Image in Z' function in the 'NIS Elements AR' software.

The resulting MIP image in Z was then analyzed on a customized pipeline on CellProfiler where nucleis and RCPs were identified, separated and quantified based on shape descriptors and distance from each identified nuclei. The pipeline was run on CellProfiler 2.1.2 and all quantification of blob and nuclei counts and intensity information was then saved as a .csv file.

Images are shown in figure 8A from two different regions inside the brain tissue sections. In the high expressing region, Neurod6 is expected to be medium-highly expressed (according to the Allen Brain atlas in situ hybridization data). In contrast, in the low expressing region, Neurod6 is expected to be expressed at a low level.

Corresponding results are shown in figure 8B from equivalent experiments that were done using the control probes, rather than the hairpin probes of the present invention.

It is noted that most of these signals in the control experiment must be non-specific since there is almost no difference between low and high expressed region. Moreover, it is noted that there are a lot of signals outside cells, which are most likely attributed to sticking of control primers non-specifically to the tissue section and generating a RCA product everywhere it sticks. Hairpin probes generate fewer signals, but those signals are most likely specific as we can see a clear difference in low expressed region and high expressed region of the tissue (as would be expected). Moreover there are very few non-specific signals outside the cells.

## Claims

1. A method for detection of a target RNA in a sample comprising:
(a) contacting said sample with at least one target-specific hairpin probe comprising:
(i) a first domain which comprises a first region of complementarity to a target sequence in said target RNA and is capable of hybridising to said target RNA; and
(ii) a second domain which is not capable of hybridising to the target RNA, which comprises a second region of complementarity to a cognate circular or circularisable probe, and which is capable of acting as a primer for an RCA reaction;
wherein the second domain is at least partly comprised within a hairpin structure of the hairpin probe, such that the second domain is not capable of binding to said cognate circular or circularisable probe and/or priming an RCA reaction until released from said hairpin structure, and
allowing said hairpin probe to hybridise to the target RNA, wherein hybridization of said first domain to said target sequence causes the hairpin structure to open and release said second domain;
(b) contacting said sample with the circular or circularisable probe cognate for said hairpin probe,
wherein the circular or circularisable probe comprises one or more regions complementary to the second region of complementarity in the second domain, and
hybridising the circular or circularisable probe to the hairpin probe at said second domain, optionally wherein the circularisable probe comprises 3' and 5' end regions complementary to the second region of complementarity in the second domain such that said ends are hybridised in juxtaposition for ligation, directly or indirectly, to each other;
(c) performing an RCA reaction using the circular probe or an RCA reaction template provided by circularising the circularisable probe; and
(d) detecting a product of said RCA reaction.

2. The method of claim 1, further comprising prior to step (c), a step of ligating, directly or indirectly, the ends of the circularisable probe to circularise the circularisable probe, thereby providing the RCA reaction template.

3. The method of claim 1 or claim 2, wherein:
(i) step (a) comprises contacting said sample with a probe set comprising two or more hairpin probes, each specific for a different target sequence in said target RNA, and wherein the first domain of each hairpin probe in the probe set comprises a region of complementarity to a different target sequence, optionally wherein within a probe set, the circular or circularisable probe for each different target sequence in a given target RNA comprises a different reporter domain or the same reporter domain; and/or
(ii) the method is for detecting more than one target RNA in a sample, wherein the sample is contacted with multiple different hairpin probes or probe sets, each specific for a different target RNA, optionally wherein the circular or circularisable probes for each RNA target comprise a different reporter domain.

4. The method of any one of claims 1 to 3, wherein:
the hairpin probe is an oligonucleotide, wherein at least part of the region at the 5' end of the oligonucleotide is capable of hybridising to a region at the 3' end of the oligonucleotide, such that the oligonucleotide forms a hairpin structure; and
the hairpin structure comprises 5' to 3';
(i) a single stranded region at the 5' end;
(ii) a first stem strand region;
(iii) a single stranded loop region; and
(iv) a second stem strand region at the 3' end.

5. The method of claim 4, wherein:
the first domain comprises all or a portion of the single stranded region at the 5' end and at least a portion of the first stem strand region of the hairpin probe, and wherein the single stranded region at the 5' end acts as a toehold for binding of the target RNA;
the second domain comprises at least a portion of the single stranded loop region; and/or
the second domain comprises at least a portion of the second stem strand region at the 3' end of the hairpin probe.

6. The method of any one of claims 1 to 3, wherein:
the hairpin probe is an oligonucleotide, wherein at least part of the region at the 5' end of the oligonucleotide is capable of hybridising to a region at the 3' end of the oligonucleotide, such that the oligonucleotide forms a hairpin structure; and
the hairpin structure comprises from 5' to 3';
(i) a first stem strand region at the 5' end;
(ii) a single stranded loop region; and
(iii) a second stem strand region at the 3' end.

7. The method of claim 6, wherein the first domain lies in the single stranded loop region of the hairpin structure and the second domain lies in the second stem strand region of the hairpin structure.

8. The method of any one of claims 1 to 7, wherein the stem of the hairpin structure comprises one or more mismatches.

9. The method of any one of claims 2 to 8, wherein:
step (b) comprises contacting said sample with two or more different circular or circularisable probes, each circular or circularisable probe being cognate for a different hairpin probe; and/or
the second domain of each member of a hairpin probe set is the same, optionally wherein step (b) comprises contacting said sample with two or more circular or circularisable probes, each circular or circularisable probe being cognate for a different set of hairpin probes.

10. The method of any one of claims 1 to 9, wherein:
the target RNA is mRNA; and/or
the sample is a cell or tissue sample.

11. The method of any one of claims 1 to 10, wherein the RCA reaction is primed by the second domain.

12. The method of any one of claims 1 to 11, wherein:
(i) the RCA product is detected by using a detection oligonucleotide which hybridises specifically to the RCA product, by using a nucleic acid stain, or by using labelled nucleotides for incorporation into the RCA product, optionally wherein the detection oligonucleotide is labelled with a directly or indirectly detectable label; and/or
(ii) the RCA product is detected *in situ* in the sample.

13. A hairpin probe for use in detecting a target RNA, wherein said hairpin probe is a target-specific hairpin probe comprising:
(i) a first domain which comprises a first region of complementarity to a target sequence in said target RNA and is capable of hybridising to said target RNA;
(ii) a second domain which is not capable of hybridising to the target RNA, which comprises a second region of complementarity to a cognate circular or circularisable probe, and which is capable of acting as a primer for an RCA reaction;
wherein the second domain is at least partly comprised within a hairpin structure of the hairpin probe, such that the second domain is not capable of binding to said cognate circular or circularisable probe and/or priming an RCA reaction until released from said hairpin structure, and wherein the hairpin structure is such that hybridisation of said first domain to said target sequence causes the hairpin structure to open and release said second domain.

14. A probe set comprising two or more target-specific hairpin probes as defined in claim 13, each hairpin probe being specific for a different target sequence in the same target RNA, wherein the first domain of each hairpin probe in the set comprises a region of complementarity to a different target sequence in the target RNA.

15. A kit comprising:
(i) a target-specific hairpin probe as defined in claim 13, or a probe set as defined in claim 14; and
(ii) a circular or circularisable probe cognate for said hairpin probe, or circular or circularisable probes cognate for the hairpin probes in the probe set, each circular or circularisable probe comprising one or more regions complementary to the second region of complementarity in the second domain of said hairpin probe(s), optionally wherein the circularisable probe-comprises 3' and 5' end regions complementary to the second region of complementarity in the second domain, such that said ends can hybridise in juxtaposition for ligation, directly or indirectly, to each other.

## Patentansprüche

1. Verfahren für ein Nachweisen einer Ziel-RNA in einer Probe, umfassend:
(a) Inkontaktbringen der Probe mit mindestens einer zielspezifischen Haarnadelsonde, umfassend:
(i) eine erste Domäne, die eine erste Region der Komplementarität zu einer Zielsequenz in der Ziel-RNA umfasst und in der Lage ist, an die Ziel-RNA zu hybridisieren; und
(ii) eine zweite Domäne, die nicht in der Lage ist, an die Ziel-RNA zu hybridisieren, die eine zweite Region der Komplementarität zu einer stammverwandten zirkulären oder zirkularisierbaren Sonde umfasst und die in der Lage ist, als Primer für eine RCA-Reaktion zu wirken;
wobei die zweite Domäne mindestens teilweise innerhalb einer Haarnadelstruktur der Haarnadelsonde umfasst ist, sodass die zweite Domäne nicht in der Lage ist, an die stammverwandte zirkuläre oder zirkularisierbare Sonde zu binden und/oder eine RCA-Reaktion vorzubereiten, bis sie aus der Haarnadelstruktur freigesetzt wird, und
Ermöglichen einer Hybridisierung der Haarnadelsonde an die Ziel-RNA, wobei die Hybridisierung der ersten Domäne an die Zielsequenz bewirkt, dass sich die Haarnadelstruktur öffnet und die zweite Domäne freisetzt;
(b) Inkontaktbringen der Probe mit der zirkulären oder zirkularisierbaren Sonde, die mit der Haarnadelsonde stammverwandt ist,
wobei die zirkuläre oder zirkularisierbare Sonde eine oder mehrere Regionen umfasst, die komplementär zu der zweiten Region der Komplementarität in der zweiten Domäne sind, und
Hybridisieren der zirkulären oder zirkularisierbaren Sonde an die Haarnadelsonde an der zweiten Domäne, wobei optional die zirkularisierbare Sonde 3'- und 5'-Endregionen umfasst, die komplementär zu der zweiten Region der Komplementarität in der zweiten Domäne sind, sodass die Enden in Nebeneinanderlage für eine Ligation direkt oder indirekt aneinander hybridisiert werden;
(c) Durchführen einer RCA-Reaktion unter Verwendung der zirkulären Sonde oder einer RCA-Reaktionsmatrize, die durch Zirkularisieren der zirkularisierbaren Sonde bereitgestellt wird; und
(d) Nachweisen eines Produktes der RCA-Reaktion.

2. Verfahren nach Anspruch 1, ferner umfassend vor Schritt (c) einen Schritt des direkten oder indirekten Ligierens der Enden der zirkularisierbaren Sonde, um die zirkularisierbare Sonde zu zirkularisieren, wodurch die RCA-Reaktionsmatrize bereitgestellt wird.

3. Verfahren nach Anspruch 1 oder 2, wobei:
(i) Schritt (a) das Inkontaktbringen der Probe mit einem Sondensatz, umfassend zwei oder mehr Haarnadelsonden, die jeweils spezifisch für eine unterschiedliche Zielsequenz in der Ziel-RNA sind, umfasst und wobei die erste Domäne jeder Haarnadelsonde in dem Sondensatz eine Region der Komplementarität zu einer unterschiedlichen Zielsequenz umfasst, optional wobei innerhalb eines Sondensatzes die zirkuläre oder zirkularisierbare Sonde für jede unterschiedliche Zielsequenz in einer gegebenen Ziel-RNA eine unterschiedliche Reporterdomäne oder die gleiche Reporterdomäne umfasst; und/oder
(ii) das Verfahren für ein Nachweisen von mehr als einer Ziel-RNA in einer Probe, wobei die Probe mit mehreren unterschiedlichen Haarnadelsonden oder Sondensätzen, die jeweils für eine unterschiedliche Ziel-RNA spezifisch sind, in Kontakt gebracht wird, optional wobei die zirkulären oder zirkularisierbaren Sonden für jede RNA-Zielsequenz eine unterschiedliche Reporterdomäne umfassen.

4. Verfahren nach einem der Ansprüche 1 bis 3, wobei:
die Haarnadelsonde ein Oligonukleotid ist, wobei mindestens ein Abschnitt der Region an dem 5'-Ende des Oligonukleotids in der Lage ist, an eine Region an dem 3'-Ende des Oligonukleotids zu hybridisieren, sodass das Oligonukleotid eine Haarnadelstruktur bildet; und
die Haarnadelstruktur 5' bis 3' umfasst;
(i) eine einzelsträngige Region an dem 5'-Ende;
(ii) eine erste Stammstrangregion;
(iii) eine einzelsträngige Schleifenregion; und
(iv) eine zweite Stammstrangregion an dem 3'-Ende.

5. Verfahren nach Anspruch 4, wobei:
die erste Domäne die gesamte oder einen Abschnitt der einzelsträngigen Region an dem 5'-Ende und mindestens einen Abschnitt der ersten Stammstrangregion der Haarnadelsonde umfasst, und wobei die einzelsträngige Region an dem 5'-Ende als Ansatzstelle für ein Binden der Ziel-RNA dient;
die zweite Domäne mindestens einen Abschnitt der einzelsträngigen Schleifenregion umfasst; und/oder
die zweite Domäne mindestens einen Abschnitt der zweiten Stammstrangregion an dem 3'-Ende der Haarnadelsonde umfasst.

6. Verfahren nach einem der Ansprüche 1 bis 3, wobei:
die Haarnadelsonde ein Oligonukleotid ist, wobei mindestens ein Abschnitt der Region an dem 5'-Ende des Oligonukleotids in der Lage ist, an eine Region an dem 3'-Ende des Oligonukleotids zu hybridisieren, sodass das Oligonukleotid eine Haarnadelstruktur bildet; und
die Haarnadelstruktur von 5' bis 3' umfasst;
(i) eine erste Stammstrangregion an dem 5'-Ende;
(ii) eine einzelsträngige Schleifenregion; und
(iii) eine zweite Stammstrangregion an dem 3'-Ende.

7. Verfahren nach Anspruch 6, wobei die erste Domäne in der einzelsträngigen Schleifenregion der Haarnadelstruktur liegt und die zweite Domäne in der zweiten Stammstrangregion der Haarnadelstruktur liegt.

8. Verfahren nach einem der Ansprüche 1 bis 7, wobei der Stamm der Haarnadelstruktur eine oder mehrere Fehlpaarungen aufweist.

9. Verfahren nach einem der Ansprüche 2 bis 8, wobei:
Schritt (b) ein Inkontaktbringen der Probe mit zwei oder mehr unterschiedlichen zirkulären oder zirkularisierbaren Sonden umfasst, wobei jede zirkuläre oder zirkularisierbare Sonde mit einer unterschiedlichen Haarnadelsonde stammverwandt ist; und/oder
die zweite Domäne jedes Mitglieds eines Haarnadelsondensatzes gleich ist, optional wobei Schritt (b) das Inkontaktbringen der Probe mit zwei oder mehr zirkulären oder zirkularisierbaren Sonden umfasst, wobei jede zirkuläre oder zirkularisierbare Sonde mit einem unterschiedlichen Satz von Haarnadelsonden stammverwandt ist.

10. Verfahren nach einem der Ansprüche 1 bis 9, wobei:
die Ziel-RNA mRNA ist; und/oder
die Probe eine Zell- oder Gewebeprobe ist.

11. Verfahren nach einem der Ansprüche 1 bis 10, wobei die RCA-Reaktion durch die zweite Domäne vorbereitet wird.

12. Verfahren nach einem der Ansprüche 1 bis 11, wobei:
(i) das RCA-Produkt nachgewiesen wird durch Verwenden eines Nachweis-Oligonukleotids, das spezifisch an das RCA-Produkt hybridisiert, durch Verwenden eines Nukleinsäure-Farbstoffs oder durch Verwenden markierter Nukleotide für ein Einbauen in das RCA-Produkt, optional wobei das Nachweis-Oligonukleotid mit einer direkt oder indirekt nachweisbaren Markierung markiert ist; und/oder
(ii) das RCA-Produkt *in situ* in der Probe nachgewiesen wird.

13. Haarnadelsonde für ein Verwenden bei einem Nachweisen einer Ziel-RNA, wobei die Haarnadelsonde eine zielspezifische Haarnadelsonde ist, umfassend:
(i) eine erste Domäne, die eine erste Region der Komplementarität zu einer Zielsequenz in der Ziel-RNA umfasst und in der Lage ist, an die Ziel-RNA zu hybridisieren;
(ii) eine zweite Domäne, die nicht in der Lage ist, an die Ziel-RNA zu hybridisieren, die eine zweite Region der Komplementarität zu einer stammverwandten zirkulären oder zirkularisierbaren Sonde umfasst und die in der Lage ist, als Primer für eine RCA-Reaktion zu wirken;
wobei die zweite Domäne mindestens teilweise innerhalb einer Haarnadelstruktur der Haarnadelsonde umfasst ist, sodass die zweite Domäne nicht in der Lage ist, an die stammverwandte zirkuläre oder zirkularisierbare Sonde zu binden und/oder eine RCA-Reaktion vorzubereiten, bis sie aus der Haarnadelstruktur freigesetzt wird, und wobei die Haarnadelstruktur derart ist, dass eine Hybridisierung der ersten Domäne an die Zielsequenz bewirkt, dass sich die Haarnadelstruktur öffnet und die zweite Domäne freisetzt.

14. Sondensatz, umfassend zwei oder mehr zielspezifische Haarnadelsonden, wie in Anspruch 13 definiert, wobei jede Haarnadelsonde für eine unterschiedliche Zielsequenz in derselben Ziel-RNA spezifisch ist, wobei die erste Domäne jeder Haarnadelsonde in dem Satz eine Region der Komplementarität zu einer unterschiedlichen Zielsequenz in der Ziel-RNA umfasst.

15. Kit, umfassend:
(i) eine zielspezifische Haarnadelsonde nach Anspruch 13 oder ein Sondensatz nach Anspruch 14; und
(ii) eine zirkuläre oder zirkularisierbare Sonde, die mit der Haarnadelsonde stammverwandt ist, oder zirkuläre oder zirkularisierbare Sonden, die mit den Haarnadelsonden in dem Sondensatz stammverwandt sind, jede zirkuläre oder zirkularisierbare Sonde eine oder mehrere Regionen umfassend, die zu der zweiten Region der Komplementarität in der zweiten Domäne der Haarnadelsonde(n) komplementär sind, optional wobei die zirkularisierbare Sonde 3'- und 5'-Endregionen umfasst, die komplementär zu der zweiten Region der Komplementarität in der zweiten Domäne sind, sodass die Enden in Nebeneinanderlage für eine Ligation direkt oder indirekt aneinander hybridisieren können.

## Revendications

1. Procédé de détection d'un ARN cible dans un échantillon comprenant :
(a) la mise en contact dudit échantillon avec l'au moins une sonde en épingle à cheveux spécifique de cible comprenant :
(i) un premier domaine qui comprend une première région de complémentarité à une séquence cible dans ledit ARN cible et est capable de s'hybrider audit ARN cible ; et
(ii) un deuxième domaine qui n'est pas capable de s'hybrider à l'ARN cible, qui comprend une deuxième région de complémentarité à une sonde apparentée circulaire ou circularisable, et qui est capable d'agir comme une amorce pour une réaction RCA ;
dans lequel le deuxième domaine est l'au moins partiellement compris au sein d'une structure en épingle à cheveux de la sonde en épingle à cheveux, de sorte que le deuxième domaine n'est pas capable de se lier à ladite sonde apparentée circulaire ou circularisable et/ou d'amorcer une réaction RCA jusqu'à ce qu'il soit libéré de ladite structure en épingle à cheveux, et
le fait de permettre à ladite sonde en épingle à cheveux de s'hybrider à l'ARN cible, dans lequel l'hybridation dudit premier domaine à ladite séquence cible provoque l'ouverture de la structure en épingle à cheveux et la libération dudit deuxième domaine ;
(b) la mise en contact dudit échantillon avec la sonde apparentée circulaire ou circularisable pour ladite sonde en épingle à cheveux,
dans lequel la sonde circulaire ou circularisable comprend une ou plusieurs régions complémentaires de la deuxième région de complémentarité dans le deuxième domaine, et
l'hybridation de la sonde circulaire ou circularisable à la sonde en épingle à cheveux au niveau dudit deuxième domaine, dans lequel facultativement la sonde circularisable comprend des régions d'extrémité 3' et 5' complémentaires de la deuxième région de complémentarité dans le deuxième domaine de sorte que lesdites extrémités sont hybridées en juxtaposition pour ligature, directement ou indirectement, l'une à l'autre ;
(c) la réalisation d'une réaction RCA en utilisant la sonde circulaire ou un modèle de réaction RCA fourni par la circularisation de la sonde circularisable ; et
(d) la détection d'un produit de ladite réaction RCA.

2. Procédé selon la revendication 1, comprenant en outre, avant l'étape (c), une étape de ligature, directement ou indirectement, des extrémités de la sonde circularisable pour circulariser la sonde circularisable, fournissant ainsi le modèle de réaction RCA.

3. Procédé selon la revendication 1 ou la revendication 2, dans lequel :
(i) l'étape (a) comprend la mise en contact dudit échantillon avec un ensemble de sondes comprenant deux sondes en épingle à cheveux ou plus, chacun spécifique d'une séquence cible différente dans ledit ARN cible, et dans lequel le premier domaine de chaque sonde en épingle à cheveux dans l'ensemble de sondes comprend une région de complémentarité à une séquence cible différente, facultativement dans lequel au sein d'un ensemble de sondes, la sonde circulaire ou circularisable pour chaque séquence cible différente dans un ARN cible donné comprend un domaine rapporteur différent ou le même domaine rapporteur ; et/ou
(ii) le procédé est destiné à détecter plus d'un ARN cible dans un échantillon, dans lequel l'échantillon est mis en contact avec de multiples sondes en épingle à cheveux différentes ou ensembles de sondes, chacune étant spécifique d'un ARN cible différent, facultativement dans lequel les sondes circulaires ou circularisables pour chaque cible d'ARN comprennent un domaine rapporteur différent.

4. Procédé selon l'une quelconque des revendications 1 à 3, dans lequel :
la sonde en épingle à cheveux est un oligonucléotide, dans lequel au moins une partie de la région à l'extrémité 5' de l'oligonucléotide est capable de s'hybrider à une région à l'extrémité 3' de l'oligonucléotide, de sorte que l'oligonucléotide forme une structure en épingle à cheveux ; et
la structure en épingle à cheveux comprend 5' à 3' ;
(i) une région simple brin à l'extrémité 5' ;
(ii) une première région de brin de tige ;
(iii) une région de boucle simple brin ; et
(iv) une deuxième région de brin de tige à l'extrémité 3' .

5. Procédé selon la revendication 4, dans lequel :
le premier domaine comprend la totalité ou une portion de la région simple brin au niveau de l'extrémité 5' et l'au moins une portion de la première région de brin de tige de la sonde en épingle à cheveux, et dans lequel la région simple brin au niveau de l'extrémité 5' agit comme un ancrage « toehold » pour la liaison de l'ARN cible ;
le deuxième domaine comprend l'au moins une portion de la région de boucle simple brin ; et/ou
le deuxième domaine comprend l'au moins une portion de la deuxième région de brin de tige à l'extrémité 3' de la sonde en épingle à cheveux.

6. Procédé selon l'une quelconque des revendications 1 à 3, dans lequel :
la sonde en épingle à cheveux est un oligonucléotide, dans lequel au moins une partie de la région à l'extrémité 5' de l'oligonucléotide est capable de s'hybrider à une région à l'extrémité 3' de l'oligonucléotide, de sorte que l'oligonucléotide forme une structure en épingle à cheveux ; et
la structure en épingle à cheveux comprend de 5' à 3' ;
(i) une première région de brin de tige à l'extrémité 5' ;
(ii) une région de boucle simple brin ; et
(iii) une deuxième région de brin de tige à l'extrémité 3'.

7. Procédé selon la revendication 6, dans lequel le premier domaine se trouve dans la région de boucle simple brin de la structure en épingle à cheveux et le deuxième domaine se trouve dans la deuxième région de brin de tige de la structure en épingle à cheveux.

8. Procédé selon l'une quelconque des revendications 1 à 7, dans lequel la tige de la structure en épingle à cheveux comprend un ou plusieurs mésappariements.

9. Procédé selon l'une quelconque des revendications 2 à 8, dans lequel :
l'étape (b) comprend la mise en contact dudit échantillon avec deux ou plus sondes circulaires ou circularisables différentes, chaque sonde circulaire ou circularisable étant apparentée à une sonde en épingle à cheveux différente ; et/ou
le deuxième domaine de chaque membre d'un ensemble de sondes en épingle à cheveux est le même, facultativement dans lequel l'étape (b) comprend la mise en contact dudit échantillon avec deux ou plus sondes circulaires ou circularisables, chaque sonde circulaire ou circularisable étant apparentée à un ensemble différent de sondes en épingle à cheveux.

10. Procédé selon l'une quelconque des revendications 1 à 9, dans lequel :
l'ARN cible est un ARNm ; et/ou
l'échantillon est un échantillon de cellules ou de tissu.

11. Procédé selon l'une quelconque des revendications 1 à 10, dans lequel la réaction RCA est amorcée par le deuxième domaine.

12. Procédé selon l'une quelconque des revendications 1 à 11, dans lequel :
(i) le produit RCA est détecté en utilisant un oligonucléotide de détection qui s'hybride spécifiquement au produit RCA, en utilisant un colorant d'acide nucléique, ou en utilisant des nucléotides marqués pour incorporation dans le produit RCA, dans lequel facultativement l'oligonucléotide de détection est marqué avec un marqueur détectable directement ou indirectement ; et/ou
(ii) le produit RCA est détecté *in situ* dans l'échantillon.

13. Sonde en épingle à cheveux destinée à être utilisée dans la détection d'un ARN cible, dans laquelle ladite sonde en épingle à cheveux est une sonde en épingle à cheveux spécifique de cible comprenant :
(i) un premier domaine qui comprend une première région de complémentarité à une séquence cible dans ledit ARN cible et est capable de s'hybrider audit ARN cible ;
(ii) un deuxième domaine qui n'est pas capable de s'hybrider à l'ARN cible, qui comprend une deuxième région de complémentarité à une sonde apparentée circulaire ou circularisable, et qui est capable d'agir comme une amorce pour une réaction RCA ;
dans lequel le deuxième domaine est au moins partiellement compris au sein d'une structure en épingle à cheveux de la sonde en épingle à cheveux, de sorte que le deuxième domaine n'est pas capable de se lier à ladite sonde apparentée circulaire ou circularisable et/ou d'amorcer une réaction RCA jusqu'à ce qu'il soit libéré de ladite structure en épingle à cheveux, et dans lequel la structure en épingle à cheveux est telle que l'hybridation dudit premier domaine à ladite séquence cible provoque l'ouverture de la structure en épingle à cheveux et la libération dudit deuxième domaine.

14. Ensemble de sondes comprenant deux ou plus sondes en épingle à cheveux spécifiques de cible telles que définies dans la revendication 13, chaque sonde en épingle à cheveux étant spécifique d'une séquence cible différente dans le même ARN cible, dans lequel le premier domaine de chaque sonde en épingle à cheveux de l'ensemble comprend une région de complémentarité à une séquence cible différente dans l'ARN cible.

15. Kit, comprenant :
(i) une sonde en épingle à cheveux spécifique de cible telle que définie dans la revendication 13, ou un ensemble de sondes tel que défini dans la revendication 14 ; et
(ii) une sonde circulaire ou circularisable apparentée à ladite sonde en épingle à cheveux, ou des sondes circulaires ou circularisables apparentées aux sondes en épingle à cheveux dans l'ensemble de sondes, chaque sonde circulaire ou circularisable comprenant une ou plusieurs régions complémentaires de la deuxième région de complémentarité dans le deuxième domaine de ladite ou desdites sonde(s) en épingle à cheveux, facultativement dans lequel la sonde circularisable-comprend des régions d'extrémité 3' et 5' complémentaires de la deuxième région de complémentarité dans le deuxième domaine, de sorte que lesdites extrémités peuvent s'hybrider en juxtaposition pour ligature, directement ou indirectement, l'une à l'autre.
